(19) Europäisches Patentamt
European Patent Office
Office européen des brevets

(11) EP 1 992 331 B1

(12) FASCICULE DE BREVET EUROPEEN

(45) Date de publication et mention de la délivrance du brevet:
**02.10.2013 Bulletin 2013/40**

(51) Int Cl.:
*A61K 8/49* (2006.01) *A61Q 5/10* (2006.01)
*A61K 8/39* (2006.01) *A61K 8/34* (2006.01)

(21) Numéro de dépôt: **08155812.4**

(22) Date de dépôt: **07.05.2008**

(54) **Composition tinctoriale comprenant une base d'oxydation aminopyrazolopyridine, un coupleur et un polyol particulier**

Färbezusammensetzung, die eine Oxidationsbasis aus Aminopyrazolopyridin und ein spezielles assoziatives Polymer enthält

Dyeing composition comprising an aminopyrazolopyridine oxidation base, a coupler and a specific polyol

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **09.05.2007 FR 0754947**

(43) Date de publication de la demande:
**19.11.2008 Bulletin 2008/47**

(73) Titulaire: **L'Oréal**
**75008 Paris (FR)**

(72) Inventeur: **Saunier, Jean-Baptiste**
**75014, PARIS (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**EP-A- 1 733 714** **EP-A- 1 792 606**
**EP-A- 1 792 903** **WO-A-01/35917**
**WO-A-02/076416** **WO-A-02/076418**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention a pour objet une composition tinctoriale comprenant au moins une base aminopyrazolopyridine, un coupleur et un polyol particulier. L'invention a aussi pour objet l'utilisation de cette composition pour la teinture des fibres kératiniques ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est déjà connu d'utiliser des bases d'oxydation aminopyrazolopyridine pour la teinture des fibres kératiniques, notamment dans la demande de brevet FR 2 801 308. Ces bases permettent d'obtenir des nuances variées.

Par ailleurs, il est connu d'utiliser dans le domaine de la coloration des fibres kératiniques des compositions de teinture mettant en oeuvre des solvants de type polyols, notamment dans le document FR2886139.

Le but de la présente invention est d'obtenir une composition pour la coloration des cheveux qui présente des propriétés tinctoriales améliorées en terme de puissance, de sélectivité et de résistance aux agents extérieurs.

Ce but est atteint avec la présente invention qui a pour objet une composition de coloration des fibres kératiniques comprenant, dans un milieu de teinture approprié,

- une ou plusieurs bases d'oxydation aminopyrazolopyridine de formule (I) suivante :

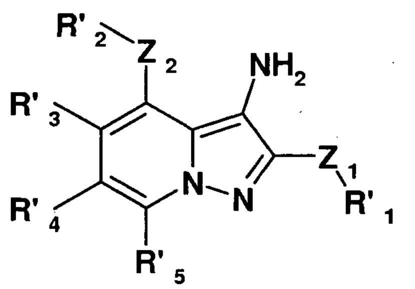

(I)

X

dans laquelle

- $Z_1$ et $Z_2$ représentent indépendamment
  - une liaison covalente simple,
  - un radical divalent choisi parmi
  - un radical $-O(CH_2)_p-$, p désignant un nombre entier allant de 0 à 6
  - un radical- $NR'_6(CH_2)_q(C_6H_4)_t-$, q désignant un nombre entier allant de 0 à 6 et t désignant 0 ou 1. $R'_6$ représente un atome d'hydrogène, un radical alkyle en $C_1$- $C_6$ éventuellement substitué par un ou plusieurs groupements hydroxy.
- $Z_1$ peut aussi représenter un radical divalent -S-, -SO-, $-SO_2-$ lorsque $R'_1$ est un radical méthyle,
- $R'_1$ et $R'_2$ représentent indépendamment:

- un hydrogène
- un radical alkyle en $C_1$-$C_{10}$, éventuellement substitué et éventuellement interrompu par un hétéroatome ou un groupement choisi parmi O, N, Si, S, SO, $SO_2$,
- un halogène,
- un radical $SO_3H$,
- un cycle de 5 à 8 chaînons, substitué ou non, saturé, insaturé ou aromatique, renfermant éventuellement un ou plusieurs hétéroatomes ou groupements choisis parmi N, O, S, $SO_2$, -CO-, le cycle pouvant être cationique et/ou substitué par un radical cationique,
- un groupement- $N^+R_{17}R_{18}R_{19}$, $R_{17}$, $R_{18}$ et $R_{19}$ étant des alkyls linéaires ou ramifiés en $C_1$- $C_5$ éventuellement substituées par un ou plusieurs groupements hydroxy.

lorsque $Z_1$ respectivement $Z_2$ représente une liaison covalente, alors R'$_1$ respectivement R'$_2$ peut aussi représenter un radical :

- alkylcarbonyle en $C_1$-$C_6$, éventuellement substitué
- -O-CO-R, -CO-O-R, NR-CO-R' ou -CO-NRR' dans lesquels R et R' représentent indépendamment un atome d'hydrogène ou un radical alkyle en $C_1$-$C_6$ éventuellement substitué,

• R'$_3$, R'$_4$ et R'$_5$, identiques ou différents, représentent

- un atome d'hydrogène,
- un radical hydroxyle,
- un radical alcoxy en $C_1$-$C_6$,
- un radical alkylthio en $C_1$-$C_6$,
- un radical amino,
- un radical monoalkylamino,
- un radical dialkylamino en $C_1$-$C_6$ dans lequel les radicaux alkyles peuvent former avec l'atome d'azote auquel ils sont rattachés un hétérocycle de 5 à 8 chaînons, saturé, insaturé, aromatique ou non, pouvant renfermer un ou plusieurs hétéroatomes ou groupements choisis parmi N, O, S, $SO_2$, CO, l'hétérocycle pouvant être cationique, et/ou substitué par un radical cationique,
- un radical alkylcarbonyle en $C_1$-$C_6$, éventuellement substitué,
- un radical -O-CO-R, -CO-O-R, NR-CO-R' ou -CO-NRR' avec R et R' tels que définis précédemment,
- un halogène,
- un radical $-NHSO_3H$,
- un radical alkyle en $C_1$-$C_4$ éventuellement substitué,
- un cycle carboné saturé, insaturé ou aromatique, éventuellement substitué.
- R'$_3$, R'$_4$ et R'$_5$, peuvent former deux à deux un cycle partiellement saturé ou non,

• X représente un ion ou groupe d'ions permettant d'assurer l'électronégativité du dérivé de formule (1),

avec la condition qu'au moins un des groupements R'$_1$ et R'$_2$ représente un radical cationique.

• un ou plusieurs coupleurs,
• un ou plusieurs polyols en $C_4$-$C_{30}$ comportant une chaîne hydrocarbonée, linéaire, ramifiée ou cyclique, saturée ou insaturée, portant au moins deux fonctions hydroxyle, ladite chaîne et ses ramifications étant éventuellement substitués par un ou plusieurs autres substituants choisis parmi les groupements carboxy, amino, halogène, aryle en $C_6$-$C_{30}$; les polyéthylène glycol à 4, 6 ou 7 motifs d'éthylène ; le dipropylène glycol.

L'invention a aussi pour objet un procédé de teinture mettant en oeuvre cette composition.
Un autre objet de l'invention est l'utilisation de la composition de la présente invention pour la teinture des fibres kératiniques, en particulier les fibres kératiniques humaines telles que les cheveux.
La composition de la présente invention permet en particulier d'obtenir une composition de coloration de fibres kératiniques qui conviennent pour une utilisation en coloration d'oxydation et permettent d'obtenir une coloration aux nuances variées, puissantes, esthétiques, peu sélectives et résistant bien aux diverses agressions que peuvent subir les cheveux tels que les shampooings, la lumière, la sueur et les déformations permanentes.
Il est à noter que dans ce qui va suivre, et à moins d'une autre indication, les bornes d'un domaine de valeurs sont comprises dans ce domaine.
Dans les composés de formule (I) ci- dessus, l'expression alkyle utilisée pour les radicaux alkyle ainsi que pour

les groupements comportant une partie alkyle, signifie une chaîne carbonée, linéaire ou ramifiée, comportant de 1 à 4 atomes de carbone, substituée ou non substituée par un ou plusieurs hétérocycles, ou par un ou plusieurs groupements phényle ou par un ou plusieurs groupes choisis parmi les atomes d'halogène tel le chlore, le brome, l'iode et le fluor ; les radicaux hydroxyle, alcoxyle, amino, carbonyle, carboxamido, sulfonyle, $-CO_2H$, $-SO_3H$, $-PO_3H_2$, $-PO_4H_2$, $-NHSO_3H$, sulfonamide, monoalkyl ($C_1$- $C_4$) amino, trialkyl ($C_1$- $C_4$) ammonium, ou bien encore par un radical dialkyl ($C_1$- $C_4$) amino dans lequel les deux groupements alkyle peuvent former, conjointement avec l'atome d'azote dudit groupement dialkyl ($C_1$- $C_4$) amino auquel ils sont liés, un cycle pouvant être interrompu par un ou plusieurs atomes d'azote, d'oxygène ou de soufre.

De même, selon l'invention, l'expression alcoxy utilisée pour les radicaux alcoxy ainsi que pour les groupements comportant une partie alcoxy, signifie une chaîne O- carbonée, linéaire ou ramifiée, comportant de 1 à 4 de carbone, substituée ou non substituée par un ou plusieurs groupes choisis parmi les hétérocycles ; les atomes d'halogène tel le chlore, le brome, l'iode et le fluor ; les radicaux hydroxyle, amino, carbonyle, carboxamido, sulfonyle, $-CO_2H$, $-SO_3H$, $-PO_3H_2$, $-PO_4H_2$, $-NHSO_3H$ sulfonamide, monoalkyl ($C_1$- $C_4$) amino, trialkyl ($C_1$- $C_4$) ammonium, ou bien encore par un radical dialkyl ($C_1$- $C_4$) amino dans lequel les deux groupements alkyle peuvent former, conjointement avec l'atome d'azote dudit groupement dialkyl ($C_1$- $C_4$) amino auquel ils sont liés, un cycle pouvant être interrompu par un ou plusieurs atomes d'azote, d'oxygène ou de soufre.

Selon l'invention, on entend par hétérocycle, un cycle aromatique ou non contenant 5, 6, 7 ou 8 sommets, et de 1 à 3 hétéroatomes choisis parmi les atomes d'azote, de soufre et d'oxygène. Ces hétérocycles peuvent être condensés sur d'autres hétérocycles ou sur un groupement phényle. Ils peuvent être substitués par un atome d'halogène ; un radical ($C_1$- $C_4$) alkyle ; un radical ($C_1$- $C_4$) alkoxy ; un radical hydroxyle ; un radical amino ; un radical ($C_1$- $C_4$) alkylamino ; di ($C_1$- $C_4$) alkylamino dans lequel les deux groupements alkyle peuvent conjointement avec l'atome d'azote auquel ils sont liés, former un cycle pouvant être interrompu par un ou plusieurs atomes d'azote, d'oxygène ou de soufre. Ces hétérocycles peuvent, en outre, être quaternisés par un radical ($C_1$- $C_4$) alkyle.

Parmi ces hétérocycles éventuellement condensés, on peut notamment citer à titre d'exemple les cycles : thiadiazole, triazole, isoxazole, oxazole, azaphosphole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine, 3- (2- hydroxyéthyl) benzothiazol- 3- ium, et 1- (2- hydroxyéthyl)- pyridinium.

Selon l'invention, on entend par phényle, un radical phényle non substitué ou substitué par un ou plusieurs radicaux cyano, carbonyle, carboxamido, sulfonyle, $-CO_2H$, $-SO_3H$, $-PO_3H_2$, $-PO_4H_2$, hydroxyle, amino, monoalkyl ($C_1$- $C_4$) amino, ou dialkyl ($C_1$- $C_4$) amino dans lequel les deux groupements alkyle peuvent former, conjointement avec l'atome d'azote dudit groupement dialkyl ($C_1$- $C_4$) amino auquel ils sont liés, un cycle pouvant être interrompu par un ou plusieurs atomes d'azote, d'oxygène ou de soufre.

Par cycle ou hétérocycle cationique, on entend un cycle contenant un ou plusieurs groupements ammoniums quaternaires.

A titre d'exemple de radicaux du type- $N^+R_{17}R_{18}R_{19}$, on peut citer les radicaux triméthylammonium, triéthylammonium, diméthyl- éthyl ammonium, diéthyl- méthylammonium, diisopropylméthylammonium, diéthyl- propyl ammonium, betahydroxyéthyl diéthyl ammonium, di (betahydroxyethyl) methylammonium, tri (betahydroxyethyl) ammonium.

A titre d'exemple d'hétérocycle cationique, on peut citer les hétérocycles imidazoliums, pyridiniums, pipéraziniums, pyrrolidiniums, morpholiniums, pyrimidiniums, thiazoliums, benzimidazoliums, benzothiazoliums, oxazoliums, benzotriazoliums, pyrazoliums, triazoliums, benzoxazoliums.

Les composés de formule (I) peuvent être éventuellement salifiés par des acides minéraux forts tels que par exemple HCl, HBr, HI, $H_2SO_4$, $H_3PO_4$, ou des acides organiques tels que, par exemple, l'acide acétique, lactique, tartrique, citrique ou succinique, benzènesulfonique, para-toluènesulfonique, formique, méthanesulfonique.

S'ils possèdent des groupements anioniques tels que les groupements $-CO_2H$, $-SO_3H$ $-PO_3H_2$, $-PO_4H_2$, les composés de formules (I) peuvent être salifiés par des hydroxydes de métaux alcalins ou alcalinoterreux tels que la soude ou la potasse, par l'ammoniaque, par les amines organiques.

Ils peuvent aussi être sous forme de solvates par exemple un hydrate ou un solvate d'alcool linéaire ou ramifié tel que l'éthanol ou l'isopropanol.

Dans le cadre de l'invention, on entend par dérivé de formule (I) toutes formes mésomères ou isomères.

A titre d'exemples de dérivés de formule (I), on peut citer les composés suivants dans lesquels $X^-$ est tel que défini précédemment :

| |
|---|
| 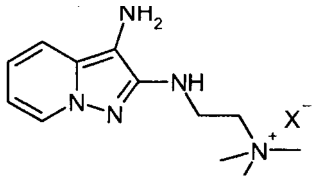<br>Sel de [2-(3-Amino-pyrazolo[1,5-a]pyridin-2-ylamino)-ethyl]-trimethyl-ammonium |
| 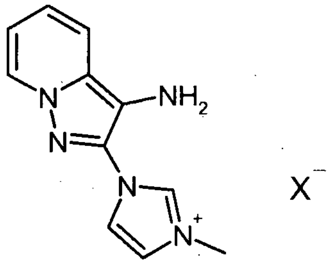<br>Sel de 3-(3-Amino-pyrazolo[1,5-a]pyridin-2-yl)-1-methyl-3H-imidazol-1-ium |
| 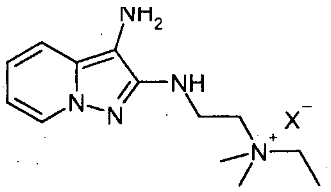<br>Sel de [2-(3-Amino-pyrazolo[1,5-a]pyridin-2-ylamino)-ethyl]-ethyl-dimethyl-ammonium |
| 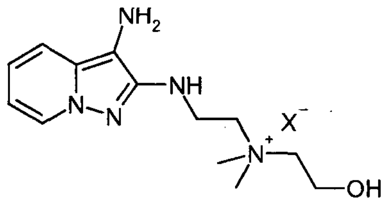<br>Sel de [2-(3-Amino-pyrazolo[1,5-a]pyridin-2-ylamino)-ethyl]-(2-hydroxy-ethyl)-dimethyl-ammonium |
| 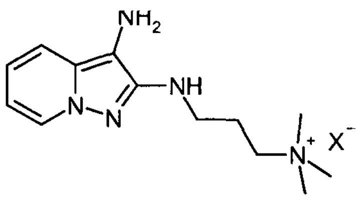<br>Sel de [3-(3-Amino-pyrazolo[1,5-a]pyridin-2-ylamino)-propyl]-trimethyl-ammonium |
| 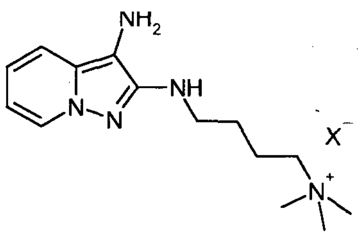<br>Sel de [4-(3-Amino-pyrazolo[1,5-a]pyridin-2-ylamino)-butyl]-trimethyl-ammonium |

(suite)

| |
|---|
| 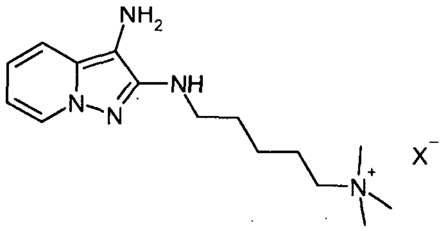 Sel de [5-(3-Amino-pyrazolo[1,5-a]pyridin-2-ylamino)-pentyl]-trimethyl-ammonium |
| 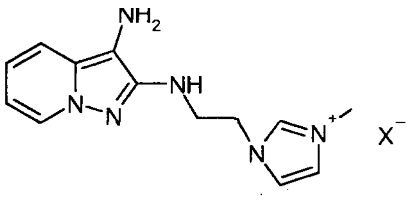 Sel de 3-[2-(3-Amino-pyrazolo[1,5-a]pyridin-2-ylamino)-ethyl]-1-methyl-3H-imidazol-1-ium |
| 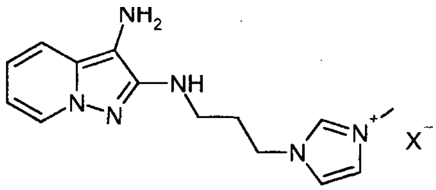 Sel de 3-[3-(3-Amino-pyrazolo[1,5-a]pyridin-2-ylamino)-propyl]-1-methyl-3H-imidazol-1-ium |
| 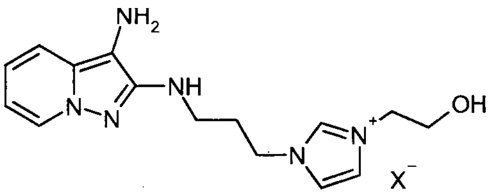 Sel de 3-[3-(3-Amino-pyrazolo[1,5-a]pyridin-2-ylamino)-propyl]-1-(2-hydroxy-ethyl)-3H-imidazol-1-ium |
| 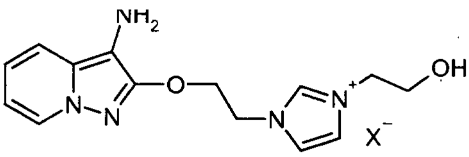 Sel de 3-[2-(3-Amino-pyrazolo[1 ,5-a]pyridin-2-yloxy)-ethyl]-1-(2-hydroxy-ethyl)-3H-imidazol-1-ium |
| 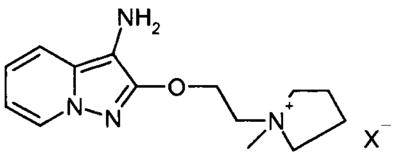 Sel de 1-{2-[(3-aminopyrazolo[1,5-a]pyridin-2-yl)oxy]ethyl}-1-methylpyrrolidinium |
| 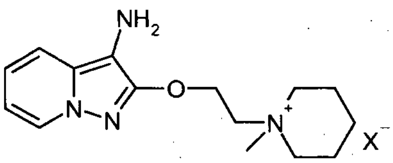 Sel de 1-{2-[(3-aminopyrazolo[1,5-a]pyridin-2-yl)oxy]ethyl}-1-methylpiperidinium |

(suite)

| |
|---|
| 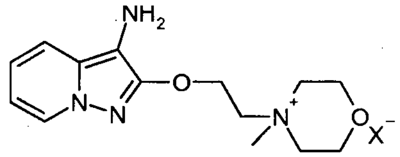<br>Sel de 4-{2-[(3-aminopyrazolo[1,5-a]pyridin-2-yl)oxy]ethyl}-4-methylmorpholin-4-ium |
| 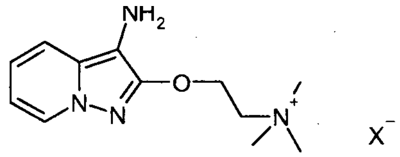<br>Sel de {2-[(3-aminopyrazolo[1,5-a]pyridin-2-yl)oxy]ethyl}- trimethyl-ammonium |
| 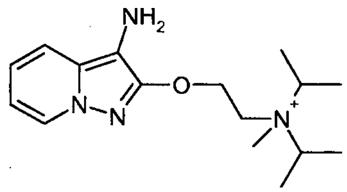<br>Sel de {2-[(3-aminopyrazolo[1,5-a]pyridin-2-yl)oxy]ethyl}-diisopropyl-methyl-ammonium |
| 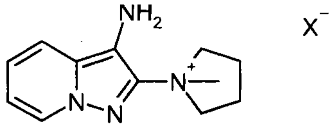<br>Sel de 1-(3-aminopyrazolo[1,5-a]pyridin-2-yl)-1-methylpyrrolidinium |
| 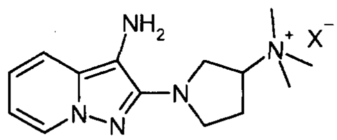<br>Sel de [1-(3-Amino-pyrazolo[1,5-a]pyridin-2-yl)-pyrrolidin-3-yl]-trimethyl-ammonium |
| 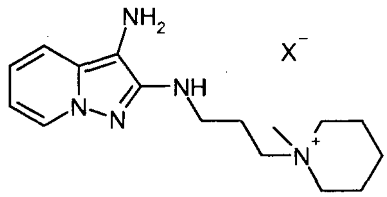<br>Sel de 1-[3-(3-Amino-pyrazolo[1,5-a]pyridin-2-ylamino)-propyl]-1-methylpiperidinium |
| 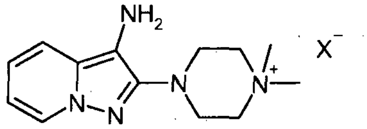<br>Sel de 4-(3-Amino-pyrazolo[1,5-a]pyridin-2-yl)-1,1-dimethyl-piperazin-1-ium |
| 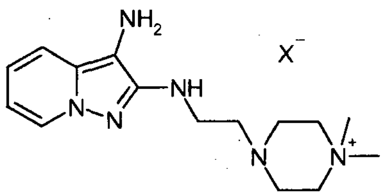 |

(suite)

| |
|---|
| Sel de 4-[2-(3-Amino-pyrazolo[1,5-a]pyridin-2-ylamino)-ethyl]-1,1-dimethyl-piperazin-1-ium |
| 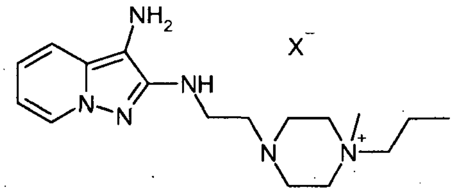<br>Sel de 4-[2-(3-Amino-pyrazolo[1,5-a]pyridin-2-ylamino)-ethyl]-1-methyl-1-propyl-piperazin-1-ium |
| 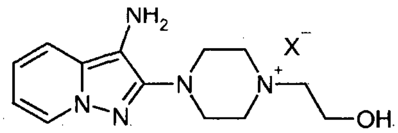<br>Sel de 4-(3-Amino-pyrazolo[1,5-a]pyridin-2-yl)-1-(2-hydroxy-ethyl)-piperazin-1-ium |
| 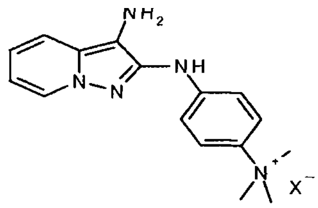<br>Sel de [4-(3-Amino-pyrazolo[1,5-a]pyridin-2-ylamino)-phenyl]-trimethyl-ammonium |
| 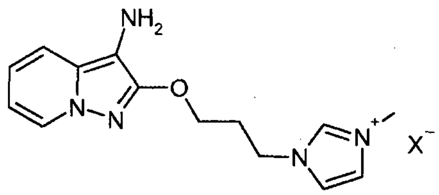<br>Sel de 3-[3-(3-Amino-pyrazolo[1,5-a]pyridin-2-yloxy)-propyl]-1-methyl-3H-imidazol-1-ium |
| 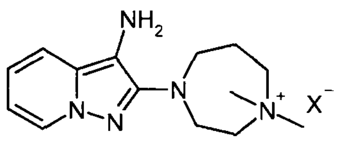<br>Sel de 4-(3-Amino-pyrazolo[1,5-a]pyridin-2-yl)-1,1-dimethyl-[1,4]diazepan-1-ium |
| 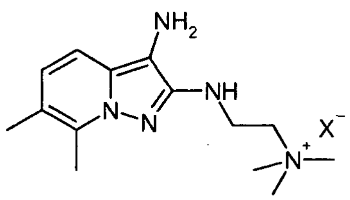<br>Sel de [2-(3-Amino-6,7-dimethyl-pyrazolo[1,5-a]pyridin-2-ylamino)-ethyl]-trimethyl-ammonium |
| 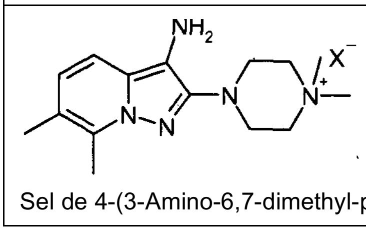<br>Sel de 4-(3-Amino-6,7-dimethyl-pyrazolo[1,5-a]pyridin-2-yl)-1,1-dimethyl-piperazin-1-ium |

(suite)

| |
|---|
| 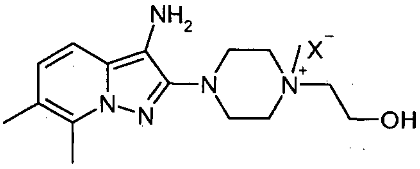<br>Sel de 4-(3-Amino-6,7-dimethyl-pyrazolo[1,5-a]pyridin-2-yl)-1-(2-hydroxy-ethyl)-1-methyl-piperazin-1-ium |
| 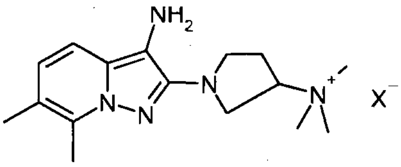<br>Sel de [1-(3-Amino-6,7-dimethyl-pyrazolo[1,5-a]pyridin-2-yl)-pyrrolidin-3-yl]-trimethyl-ammonium |
| 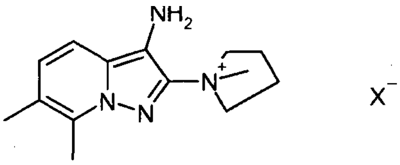<br>Sel de 1-(3-amino-6,7-dimethylpyrazolo[1,5-a]pyridin-2-yl)-1-methylpyrrolidinium |
| 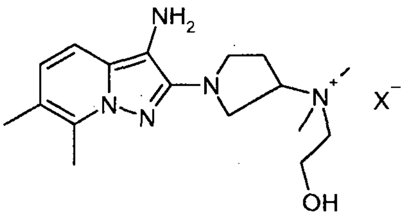<br>Sel de [1-(3-Amino-6,7-dimethyl-pyrazolo[1,5-a]pyridin-2-yl)-pyrrolidin-3-yl]-(2-hydroxy-ethyl)-dimethyl-ammonium |
| 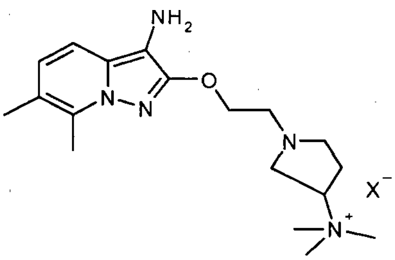<br>Sel de {1-[2-(3-Amino-6,7-dimethyl-pyrazolo[1,5-a]pyridin-2-yloxy)-ethyl]-pyrrolidin-3-yl}-trimethyl-ammonium |
| 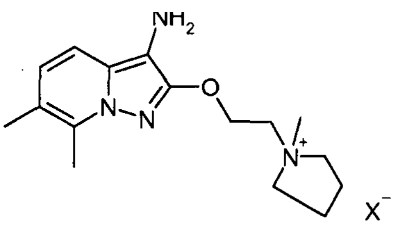<br>Sel de 1-{2-[(3-amino-6,7-dimethylpyrazolo[1,5-a]pyridin-2-yl)oxy]ethyl}-1-methyl pyrrolidinium |
| 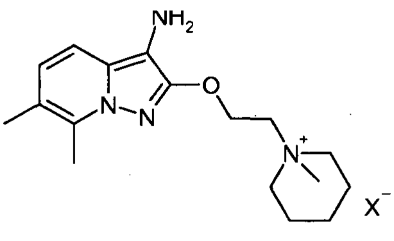 |

(suite)

| |
|---|
| Sel de 1-{2-[(3-amino-6,7-dimethylpyrazolo[1,5-a]pyridin-2-yl)oxy]ethyl}-1-methylpiperidinium |
| 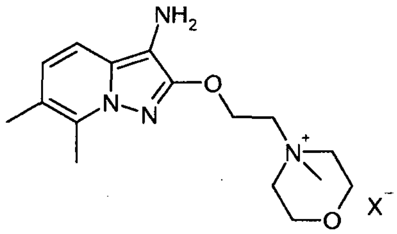 Sel de 4-{2-[(3-amino-6,7-dimethylpyrazolo[1,5-a]pyridin-2-yl)oxy]ethyl}-4-methylmorpholin-4-ium |
| 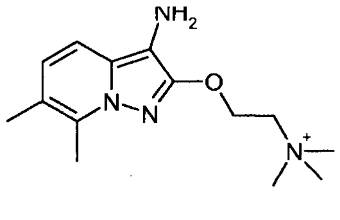 Sel de {2-[(3-amino-6,7-dimethylpyrazolo[1,5-a]pyridin-2-yl)oxy]ethyl}-trimethyl-ammonium |
| 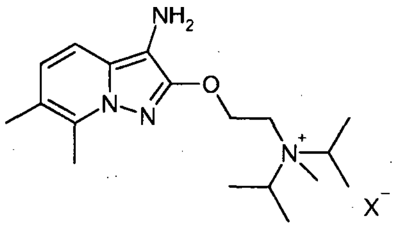 Sel de {2-[(3-amino-6,7-dimethylpyrazolo[1,5-a]pyridin-2-yl)oxy]ethyl}-diisopropyl-methyl-ammonium |
| 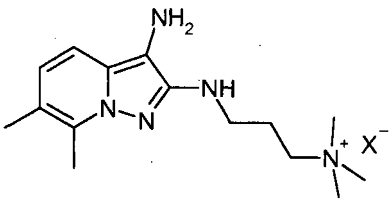 Sel de [3-(3-Amino-6,7-dimethyl-pyrazolo[1,5-a]pyridin-2-ylamino)-propyl]-trimethyl-ammonium |
| 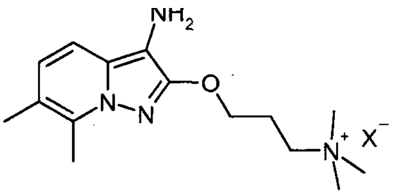 Sel de [3-(3-Amino-6,7-dimethyl-pyrazolo[1,5-a]pyridin-2-yloxy)-propyl]-trimethyl-ammonium |
| 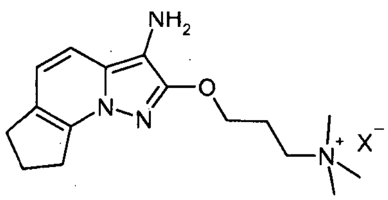 Sel de [3-(3-Amino-7,8-dihydro-6H-cyclopenta[e]pyrazolo[1,5-a]pyridin-2-yloxy)-propyl]-trimethyl-ammonium |

(suite)

| |
|---|
| 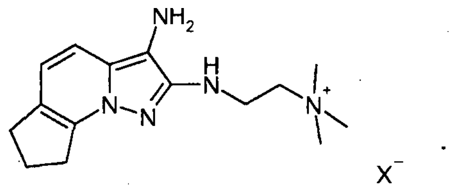<br>Sel de {2-[(3-amino-7,8-dihydro-6H-cyclopenta[e]pyrazolo[1,5-a]pyridin-2-yl)amino]-ethyl}-trimethyl-ammonium |
| 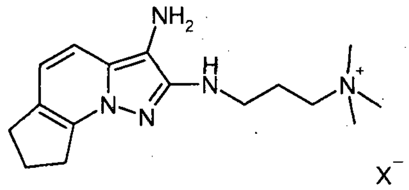<br>Sel de {3-[(3-amino-7,8-dihydro-6H-cyclopenta[e]pyrazolo[1,5-a]pyridin-2-yl)amino]-propyl}-trimethyl-ammonium |
| 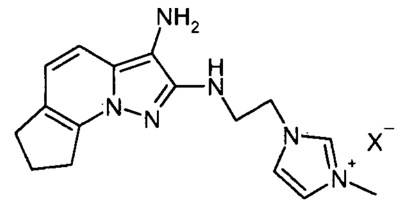<br>Sel de 1-{2-[(3-amino-7,8-dihydro-6H-cyclopenta[e]pyrazolo[1,5-a]pyridin-2-yl)amino]ethyl}-3-methyl-1 H-imidazol-3-ium |
| 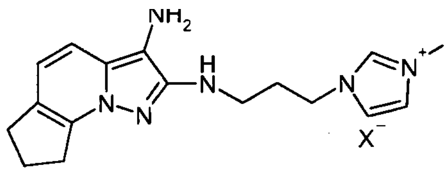<br>Sel de 1-{3-[(3-amino-7,8-dihydro-6H-cyclopenta[e]pyrazolo[1,5-a]pyridin-2-yl)amino]propyl}-3-methyl-1H-imidazol-3-ium |
| 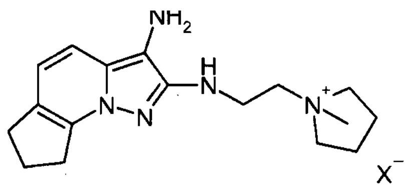<br>Sel de 1-{2-[(3-amino-7,8-dihydro-6H-cyclopenta[e]pyrazolo[1,5-a]pyridin-2-yl)amino]ethyl}-1-methylpyrrolidinium |
| 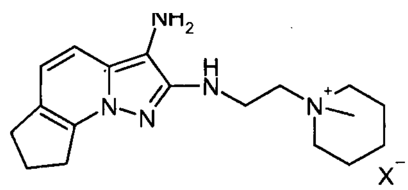<br>Sel de 1-{2-[(3-amino-7,8-dihydro-6H-cyclopenta[e]pyrazolo[1,5-a]pyridin-2-yl)amino]ethyl}-1-methylpiperidinium |
| 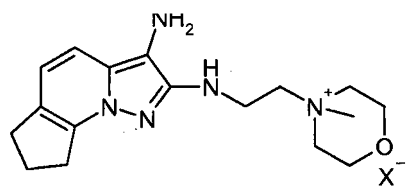 |

(suite)

| |
|---|
| Sel de 4-{2-[(3-amino-7,8-dihydro-6H-cyclopenta[e]pyrazolo[1,5-a]pyridin-2-yl)amino]ethyl}-4-methylmorpholin-4-ium |
| 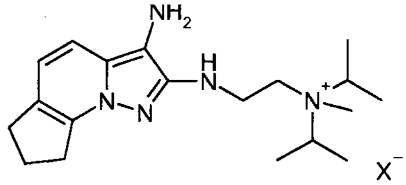<br>Sel de {2-[(3-amino-7,8-dihydro-6H-cyclopenta[e]pyrazolo[1,5-a]pyridin-2-yl)amino]ethyl}-diisopropyl-methyl-ammonium |
| 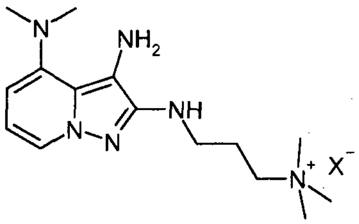<br>Sel de [3-(3-Amino-4-dimethylamino-pyrazolo[1,5-a]pyridin-2-ylamino)-propyl]-trimethyl-ammonium |
| 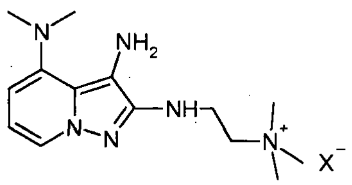<br>Sel de [2-(3-Amino-4-dimethylamino-pyrazolo[1,5-a]pyridin-2-ylamino)-ethyl]-trimethyl-ammonium |
| 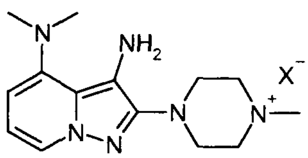<br>Sel de 4-(3-Amino-4-dimethylamino-pyrazolo[1,5-a]pyridin-2-yl)-1-methyl-piperazin-1-ium |
| 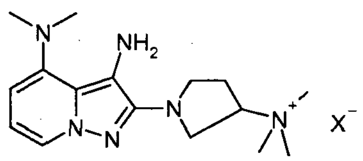<br>Sel de [1-(3-Amino-4-dimethylamino-pyrazolo[1,5-a]pyridin-2-yl)-pyrrolidin-3-yl]-trimethyl-ammonium |
| 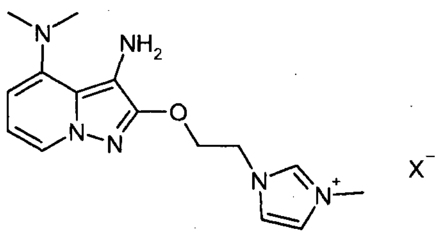<br>Sel de 3-[2-(3-Amino-4-dimethylamino-pyrazolo[1,5-a]pyridin-2-yloxy)-ethyl]-1-methyl-3H-imidazol-1-ium |

(suite)

| |
|---|
| 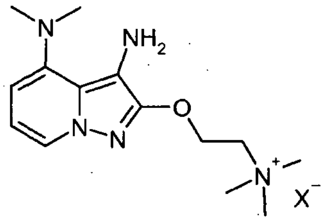<br>Sel de [2-(3-Amino-4-dimethylamino-pyrazolo[1,5-a]pyridin-2-yloxy)-ethyl]-trimethyl-ammonium |
| 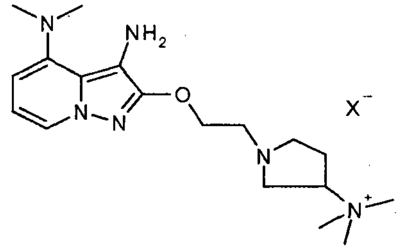<br>Sel de {1-[2-(3-Amino-4-dimethylamino-pyrazolo[1,5-a]pyridin-2-yloxy)-ethyl]-pyrrolidin-3-yl}-trimethyl-ammonium |
| 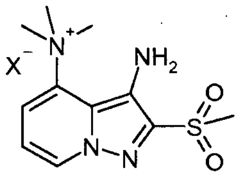<br>Sel de (3-Amino-2-methanesulfonyl-pyrazolo[1,5-a]pyridin-4-yl)-trimethyl-ammonium |
| 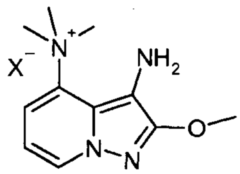<br>Sel de (3-Amino-2-methoxy-pyrazolo[1,5-a]pyridin-4-yl)-trimethyl-ammonium |

La nature du contre-ion n'est pas déterminante sur le pouvoir tinctorial des composés de formule (1).

Lorsque $R'_1$ ou $R'_2$ désignent un hétérocycle, cet hétérocycle est de préférence un hétérocycle cationique ou un hétérocycle substitué par un radical cationique. A titre d'exemple, on peut citer les imidazoles substitués par un radical ammonium quaternaire ou les imidazoliums, les pipérazines substitués par un radical ammonium quaternaire ou les pipéraziniums, les pyrrolidines substitués par un radical ammonium quaternaire ou les pyrrolidiniums, les diazépanes substitués par un radical ammonium quaternaire ou les diazépaniums.

Selon un mode de réalisation différent, $R'_1$ ou $R'_2$ représentent un groupement- $N^+R_{17}R_{18}R_{19}$, $R_{17}$, $R_{18}$ et $R_{19}$ étant des alkyls linéaires ou ramifiés en $C_1$- $C_5$ éventuellement substituées par un ou plusieurs groupements hydroxy, tel que trialkylammonium, tri (hydroxyalkyl) ammonium, hydroxyalkyl- dialkyl- ammonium ou di (hydroxyalkyl) alkylammonium.

Les radicaux $R'_3$, $R'_4$ et $R'_5$ indépendamment peuvent être un atome d'hydrogène, un radical alkyle en $C_1$-$C_4$ pouvant être substitué. A titre d'exemple, on peut citer les radicaux méthyle, éthyle, hydroxy éthyle, amino éthyle, propyle, butyle. Selon un mode de réalisation particulier, $R'_3$, $R'_4$ et $R'_5$ représentent indépendamment un atome d'hydrogène, un radical alkyle en $C_1$-$C_4$.

Selon un mode de réalisation particulier, $R'_4$ et $R'_5$ forment ensemble un cycle partiellement saturé ou insaturé à 5 ou 6 chaînons, notamment un cyclopentène ou cyclohexène, éventuellement substitué.

Selon un mode de réalisation particulier, le composé de formule (I) correspond à la formule (I') suivante :

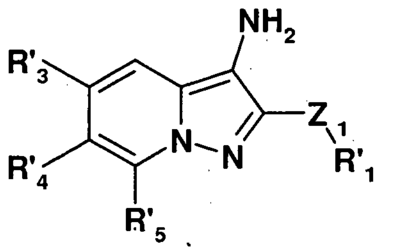

(I')

dans laquelle $Z_1$, $R'_1$, $R'_3$, $R'_4$ et $R'_5$ sont tels que définis précédemment.

Selon un mode de réalisation particulier de cette formule, $Z_1$ représente une liaison covalente, un radical- $NR'_6(CH_2)_q$- ou un radical- $O(CH_2)_p$- et $R'_1$ est un radical cationique

La ou les bases d'oxydation de l'invention sont en général présentes chacune en quantité comprise entre 0,001 à 10 % en poids environ du poids total de la composition tinctoriale, de préférence entre 0,005 et 6 %.

La composition tinctoriale selon l'invention contient un ou plusieurs coupleurs conventionnellement utilisés pour la teinture des fibres kératiniques. Parmi ces coupleurs, on peut notamment citer les méta-phénylènediamines, les méta-aminophénols, les méta-diphénols, les coupleurs naphtaléniques, les coupleurs hétérocycliques et leurs sels d'addition.

A titre d'exemple de coupleur, on peut citer le 2- méthyl 5- aminophénol, le 5- N- (β- hydroxyéthyl) amino 2-méthyl phénol, le 6- chloro- 2- méthyl- 5- aminophénol, le 3- amino phénol, le 2, 4- dichloro- 3- aminophenol, le 5- amino-4- chloro- o- cresol, le 1, 3- dihydroxy benzène, le 1, 3- dihydroxy 2- méthyl benzène, le 4- chloro 1, 3- dihydroxy benzène, le 2, 4- diamino 1- (β- hydroxyéthyloxy) benzène, le 2- amino 4- (ß- hydroxyéthylamino) 1- méthoxybenzène, le 1, 3-diamino benzène, le 1, 3- bis- (2, 4- diaminophénoxy) propane, la 3- uréido aniline, le 3- uréido 1- diméthylamino benzène, le sésamol, le 1- β- hydroxyéthylamino- 3, 4- méthylènedioxybenzène, l'α- naphtol, le 2 méthyl- 1- naphtol, le 1, 5-dihydroxy naphtalene, le 2, 7- naphthalenediol, le 1- acetoxy- 2- methylnaphthalene, le 6- hydroxy indole, le 4- hydroxy indole, le 4- hydroxy N- méthyl indole, la 2- amino- 3- hydroxy pyridine, la 6- hydroxy benzomorpholine la 3, 5- diamino-2, 6- diméthoxypyridine, les 2, 6- dihydroxy- 3- 4- dimethyl pyridine, le 3- amino- 2- methylamino- 6- methoxypyridine, le 1- N- (ß- hydroxyéthyl) amino- 3, 4- méthylène dioxybenzène, le 2, 6- bis- (ß- hydroxyéthylamino) toluène, le 3- methyl-1- phenyl- 5- pyrazolone et leurs sels d'addition avec un acide.

Dans la composition de la présente invention, le ou les coupleurs représentent en général une quantité comprise entre 0,001 et 10 % en poids environ du poids total de la composition tinctoriale, de préférence entre 0,005 et 6 %.

La composition tinctoriale de l'invention peut éventuellement comprendre une ou plusieurs bases d'oxydation additionnelle conventionnellement utilisées pour la teinture de fibres kératiniques, différentes des bases d'oxydation de formule (I).

La composition de la présente invention peut en outre comprendre une ou plusieurs bases d'oxydation additionnelles classiquement utilisées en teinture d'oxydation différentes des bases d'oxydation de formule (I) . A titre d'exemple, ces bases d'oxydation additionnelles sont choisies parmi les para- phénylènediamines autres que celles décrites précédemment, les bis- phénylalkylènediamines, les para- aminophénols, les bis- para- aminophénols, les ortho- aminophénols, les bases hétérocycliques différentes des bases d'oxydation de formule (I) et leurs sels d'addition.

Parmi les para- phénylènediamines, on peut citer à titre d'exemple, la para- phénylènediamine, la para- toluènediamine, la 2- chloro para- phénylènediamine, la 2, 3- diméthyl para- phénylènediamine, la 2, 6- diméthyl para-phénylènediamine, la 2, 6- diéthyl para- phénylènediamine, la 2, 5- diméthyl para- phénylènediamine, la N, N- diméthyl para- phénylènediamine, la N, N- diéthyl para- phénylènediamine, la N, N- dipropyl para- phénylènediamine, la 4- amino N, N- diéthyl 3- méthyl aniline, la N, N- bis- (β- hydroxyéthyl) para- phénylènediamine, la 4- N, N- bis- (β- hydroxyéthyl) amino 2- méthyl aniline, la 4- N, N- bis- (β- hydroxyéthyl) amino 2- chloro aniline, la 2- β- hydroxyéthyl para- phénylènediamine, la 2- fluoro para- phénylènediamine, la 2- isopropyl para- phénylènediamine, la N- (β- hydroxypropyl) para-phénylènediamine, la 2- hydroxyméthyl para- phénylènediamine, la N, N- diméthyl 3- méthyl para- phénylènediamine, la N, N- (éthyl, β- hydroxyéthyl) para- phénylènediamine, la N- (β, γ- dihydroxypropyl) para- phénylènediamine, la N-(4'- aminophényl) para- phénylènediamine, la N- phényl para- phénylènediamine, la 2- β- hydroxyéthyloxy para- phénylènediamine, la 2- β- acétylaminoéthyloxy para- phénylènediamine, la N- (β- méthoxyéthyl) para- phénylène- diamine, la 4- aminophénylpyrrolidine, la 2- thiényl para- phénylènediamine, le 2- β hydroxyéthylamino 5- amino toluène, la 3-hydroxy 1- (4'- aminophényl) pyrrolidine et leurs sels d'addition avec un acide.

Parmi les para- phénylènediamines citées ci- dessus, la para- phénylènediamine, la para- toluènediamine, la 2- isopropyl para- phénylènediamine, la 2- β- hydroxyéthyl para- phénylènediamine, la 2- β- hydroxyéthyloxy para-phénylène- diamine, la 2, 6- diméthyl para- phénylènediamine, la 2, 6- diéthyl para- phénylènediamine, la 2, 3- diméthyl para- phénylènediamine, la N, N- bis- (β- hydroxyéthyl) para- phénylènediamine, la 2- chloro para- phénylènediamine, la 2- β- acétylaminoéthyloxy para- phénylènediamine, et leurs sels d'addition avec un acide sont particulièrement préférées.

Parmi les bis- phénylalkylènediamines, on peut citer à titre d'exemple, le N, N'- bis- (β- hydroxyéthyl) N, N'- bis- (4'- aminophényl) 1, 3- diamino propanol, la N, N'- bis- (β- hydroxyéthyl) N, N'- bis- (4'- aminophényl) éthylènediamine, la N, N'- bis- (4- aminophényl) tétraméthylènediamine, la N, N'- bis- (β- hydroxyéthyl) N, N'- bis- (4- aminophényl) tétraméthylènediamine, la N, N'- bis- (4- méthyl- aminophényl) tétraméthylènediamine, la N, N'- bis- (éthyl) N, N'- bis- (4'- amino, 3'- méthylphényl) éthylènediamine, le 1, 8- bis- (2, 5- diamino phénoxy)- 3, 6- dioxaoctane, et leurs sels d'addition avec un acide.

Parmi les para- aminophénols, on peut citer à titre d'exemple, le para- aminophénol, le 4- amino 3- méthyl phénol, le 4- amino 3- fluoro phénol, le 4- amino 3- hydroxyméthyl phénol, le 4- amino 2- méthyl phénol, le 4- amino 2- hydroxyméthyl phénol, le 4- amino 2- méthoxyméthyl phénol, le 4- amino 2- aminométhyl phénol, le 4- amino 2- (β- hydroxyéthyl aminométhyl) phénol, le 4- amino 2- fluoro phénol, le 1- hydroxy- 4- methylamino- benzene, le 2- 2'- methylenebis- 4- amino phenol et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2, 5- diamino pyridine, la 2- (4- méthoxyphényl) amino 3- amino pyridine, la 2, 3- diamino 6- méthoxy pyridine, la 2- (β- méthoxyéthyl) amino 3- amino 6- méthoxy pyridine, la 3, 4- diamino pyridine, et leurs sels d'addition avec un acide.

Parmi les dérivés pyrimidiniques, on peut citer les composés décrits par exemple dans les brevets DE 2359399 ; JP 88- 169571 ; JP 05- 63124 ; EP 0770375 ou demande de brevet WO 96/15765 comme la 2, 4, 5, 6- tétra- aminopyrimidine, la 4- hydroxy 2, 5, 6- triaminopyrimidine, la 2- hydroxy 4, 5, 6- triaminopyrimidine, la 2, 4- dihydroxy 5, 6- diaminopyrimidine, la 2, 5, 6- triaminopyrimidine, et les dérivés pyrazolo- pyrimidiniques tels ceux mentionnés dans la demande de brevet FR- A- 2750048 et parmi lesquels on peut citer la pyrazolo- [1, 5- a]- pyrimidine- 3, 7- diamine ; la 2, 5- diméthyl pyrazolo- [1, 5- a]- pyrimidine- 3, 7- diamine ; la pyrazolo- [1, 5- a]- pyrimidine- 3, 5- diamine ; la 2, 7- diméthyl pyrazolo- [1, 5- a]- pyrimidine- 3, 5- diamine ; le 3- amino pyrazolo- [1, 5- a]- pyrimidin- 7- ol ; le 3- amino pyrazolo- [1, 5- a]- pyrimidin- 5- ol ; le 2- (3- amino pyrazolo- [1, 5- a]- pyrimidin- 7- ylamino)- éthanol, le 2- (7- amino pyrazolo- [1, 5- a]- pyrimidin- 3- ylamino)- éthanol, le 2- [(3- amino- pyrazolo [1, 5- a] pyrimidin- 7- yl)- (2- hydroxy- éthyl)- amino]- éthanol, le 2- [(7- amino- pyrazolo [1, 5- a] pyrimidin- 3- yl)- (2- hydroxy- éthyl)- amino]- éthanol, la 5, 6- diméthyl pyrazolo- [1, 5- a]- pyrimidine- 3, 7- diamine, la 2, 6- diméthyl pyrazolo- [1, 5- a]- pyrimidine- 3, 7- diamine, la 2, 5, N 7, N 7- tetraméthyl pyrazolo- [1, 5- a]- pyrimidine- 3, 7- diamine, la 3- amino- 5- méthyl- 7- imidazolylpropylamino pyrazolo- [1, 5- a]- pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR- A- 2 733 749 et DE 195 43 988 comme le 4, 5- diamino 1- méthyl pyrazole, le 4, 5- diamino 1- (β- hydroxyéthyl) pyrazole, le 3, 4- diamino pyrazole, le 4, 5- diamino 1- (4'- chlorobenzyl) pyrazole, le 4, 5- diamino 1, 3- diméthyl pyrazole, le 4, 5- diamino 3- méthyl 1- phényl pyrazole, le 4, 5- diamino 1- méthyl 3- phényl pyrazole, le 4- amino 1, 3- diméthyl 5- hydrazino pyrazole, le 1- benzyl 4, 5- diamino 3- méthyl pyrazole, le 4, 5- diamino 3- tert- butyl 1- méthyl pyrazole, le 4, 5- diamino 1- tert- butyl 3- méthyl pyrazole, le 4, 5- diamino 1- (β- hydroxyéthyl) 3- méthyl pyrazole, le 4, 5- diamino 1- éthyl 3- méthyl pyrazole, le 4, 5- diamino 1- éthyl 3- (4'- méthoxyphényl) pyrazole, le 4, 5- diamino 1- éthyl 3- hydroxyméthyl pyrazole, le 4, 5- diamino 3- hydroxyméthyl 1- méthyl pyrazole, le 4, 5- diamino 3- hydroxyméthyl 1- isopropyl pyrazole, le 4, 5- diamino 3- méthyl 1- isopropyl pyrazole, le 4- amino 5- (2'- aminoéthyl) amino 1, 3- diméthyl pyrazole, le 3, 4, 5- triamino pyrazole, le 1- méthyl 3, 4, 5- triamino pyrazole, le 3, 5- diamino 1- méthyl 4- méthylamino pyrazole, le 3, 5- diamino 4- (β- hydroxyéthyl) amino 1- méthyl pyrazole, et leurs sels d'addition avec un acide.

On peut aussi citer les diaminopyrazolinones décrites dans la demande de brevet FR2886137 et en particulier la 2, 3- diamino- 6, 7- dihydro- 1 H, 5H- pyrazol- 1- one et ses sels.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Dans le cadre de la présente invention, la composition comprend un polyol particulier.

A titre d'exemples de polyol en $C_4$- $C_{30}$, on peut citer les diols, ramifiés ou non ramifiés, tels que le 1, 4-butanediol ; le 1, 5- pentanediol ; le 1, 6- hexanediol ; le néopentylglycol (ou le 2, 3- diméthyl- 1, 3- propanediol) ; le 2, 5- hexanediol ; l'amylèneglycol (ou le 2, 4- pentanediol) ; l'hexylèneglycol (ou le 2- méthyl- 2, 4- pentanediol) ; l'isoprèneglycol (ou le 3- méthyl- 1, 3- butanediol) ; le pinacol (ou le 2, 3- diméthyl- 2, 3- butanediol) ; le 1- méthoxy- 2, 4- butanediol ; le 4- méthoxy- 1, 2- butanediol ; les triols, ramifiés ou non ramifiés, tels que le 1, 2, 4- butanetriol et le 1, 2, 6- hexanetriol ; les polyéthylène glycol à 4, 6 ou 7 motifs d'éthylène ; le dipropylène glycol.

Selon un mode de réalisation particulier de l'invention, le ou les polyols sont en $C_4$-$C_{15}$. De préférence, ils sont choisis parmi l'hexylèneglycol; le néopentylglycol ; l'isoprèneglycol.

Le ou les polyols sont présents dans la composition conforme à la présente invention dans des proportions généralement comprises entre 0,1 et 40 % en poids, et encore plus particulièrement entre 0,5 et 20 % en poids du poids total de la composition.

Le milieu approprié pour la teinture appelé aussi support de teinture comprend généralement de l'eau ou un mélange d'eau et d'au moins un monoalcool liquide hydrosoluble comme par exemple les alcanols inférieurs en $C_1$-$C_4$, tels que l'éthanol et l'isopropanol.

Ces monoalcools liquides hydrosolubles sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante :

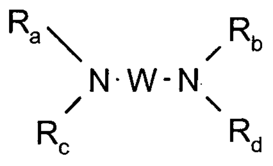

(II)

dans laquelle W est un reste propylène éventuellement substitué par un groupement hydroxyle ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, en présence d'un agent oxydant pendant un temps suffisant pour développer la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

La révélation à pH acide peut s'avérer particulièrement intéressante.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant une ou plusieurs bases d'oxydation de formule (1), un ou plusieurs coupleurs et un ou plusieurs polyols selon d'invention avec un agent oxydant, et l'application du mélange obtenu sur les fibres kératiniques pendant un temps suffisant pour développer la coloration désirée.

Les exemples qui suivent servent à illustrer l'invention sans toutefois présenter un caractère limitatif.

## EXEMPLES

## EXEMPLES DE TEINTURE

### EXEMPLE 1

La composition 1 comprend les colorants suivants (mol):

| Moles pour 100 g de composition | Chlorhydrate de chlorure de 2-[(3-aminopyrazolo [1,5-a]pyridin-2-yl)amino]-N, N, N-trimethylethanaminium | 6-Hydroxybenzomorpholine |
|---|---|---|
| Composition 1 | 0.01 | 0.01 |

introduits dans le milieu suivant :

| | |
|---|---|
| - Mélange d'alcools linéaires en $C_{18}$ à $C_{24}$ [$C_{18}$/$C_{20}$/$C_{22}$/$C_{24}$ : 7/58/30/6 - teneur en alcool > 95%] | 3 g |
| - Mélange d'alcools linéaires en $C_{18}$ à $C_{24}$ [$C_{18}$/$C_{20}$/$C_{22}$/$C_{24}$ : 7/58/30/6 - teneur en alcool > 95%] oxyéthylénés (30 OE) | 1 g |
| - Alcool stéarylique oxyéthyléné (2 OE) | 4.5 g |
| - Alcool stéarylique oxyéthyléné (21 OE) | 1.75 g |
| - Acide polyacrylique réticulé (Carbopol 980 de GOODRICH) | 0.6 g |
| - Acide oléique | 2.6 g |
| - Aculyn 44 vendu par ROHM & HAAS | 1.4 g M.A. |
| - Monoisopropanolamide d'acides de coprah | 3 g |

(suite)

| | | |
|---|---|---|
| - Polymère cationique de formule W | | 4 g M.A. |
| - Héxylène glycol | | 6 g |
| - Métabisulfite de sodium | | 0.71 g |
| - EDTA (Acide éthylènediamine tétra-acétique) | | 0.2 g |
| - Ter-butyl hydroquinone | | 0.3 g |
| - Monoéthanolamine | | 1 g |
| - Ammoniaque à 20% de NH3 | | 11 g |
| - Parfum | q.s. | |
| - Eau déminéralisée | q.s.p | 100 g |

**Mode d'application**

La composition est diluée extemporanément avec 1.5 fois son volume d'eau oxygénée à 25 volumes et dont le pH est de 3.
Le mélange ainsi réalisé est appliqué sur des cheveux gris naturels à 90% blancs, à raison de 30 g pour 3 g de cheveux pendant 30mn.
Les cheveux sont ensuite rincés, lavés avec un shampooing standard et séchés.

**Résultats**

La coloration capillaire est évaluée de manière visuelle

| | Hauteur de ton 90% BN | Reflet 90% BN |
|---|---|---|
| Composition 1 | Blond | Vert |

Ces nuances sont tenaces et uniformes.

**EXEMPLE 2**

Les compositions suivantes ont été préparées

| | | | |
|---|---|---|---|
| 1-{2-[(3-Aminopyrazolo[1,5-a]pyridin-2-yl)amino]ethyl}-3-methyl-1H-imidazol-3-ium chlorhydrate | | 1.3 g | (0.004 moles %) |
| -Méthyl 5-(2-hydroxyethyl) amino phénol | | | 0.67 g (0.004 moles %) |
| Métabisulfite de sodium | | | 0.68 g |
| Acide ascorbique | | 0.3 g | |
| Ammoniaque (à 20.5 % en NH3) | | | 10 g |
| Solvants | | 6 g | |
| Eau | Q.S.P | | 100 g |

Composition 2 : le solvant est l'éthanol (comparatif)
Composition 3 : le solvant est l'héxylène glycol

**Mode d'application**

Les compositions ont été diluées extemporanément avec 1 fois son poids en eau oxygénée à 20 volumes et dont le pH est de 3.
Le mélange ainsi réalisé a été appliqué sur des cheveux gris naturels à 90% blancs permanentés ou non, à raison de 20 g pour 2 g de cheveux pendant 30mn.
Les cheveux ont été ensuite rincés, lavés avec un shampooing standard et séchés.

**Détermination de la couleur**

La coloration des cheveux est évaluée dans le système L*a*b* system, avec un spectrophotomètre SF600X de Datacolor (diffus, 8°) : ouverture SAV, mesure en SCI, UVI avec l'illuminant D65.
Dans ce système, L représente l'intensité, plus la valeur de L* est faible, plus la coloration obtenues es intense La chromaticité est mesurée par les valeurs a* et b*, a* représentant l'axe ruge/vert et b* l'axe jaune/bleu

**Sélectivité de la coloration**

La sélectivité de la coloration est la variation de la couleur entre des cheveux naturels et des cheveux permanentés Les cheveux naturels sont représentatif de la nature des cheveux à la racine alors que les cheveux permanentés sont représentatifs de la nature des cheveux à la pointe.
La sélectivité est mesurée par :

ΔE, qui est la variation de la couleur entre les cheveux naturels et les cheveux permanents, est obtenu à partir de la formule :

$$\Delta E = \sqrt{(L^* - L_o{}^*)^2 + (a^* - a_o{}^*)^2 + (b^* - b_o{}^*)^2}$$

dans laquelle L* représente l'intensité a* et b*, la chromaticité des cheveux colorés naturels et L0* représente l'intensité et a0* et b0* la chromaticité des cheveux colorés permanenté. Plus la valeur de ΔE est faible, plus la sélectivité est faible et la coloration uniforme le long des cheveux.
Le tableau suivant regroupe les valeurs de L*a*b* mesurées sur les cheveux naturels et permanentés après coloration ainsi que le calcul de la sélectivité pour chaque composition.

| | *Après coloration sur fibre naturelle* | | | *Après coloration sur fibre permanentée* | | | *Sélectivité* |
|---|---|---|---|---|---|---|---|
| | *L** | *a** | *b** | *L** | *a** | *b** | |
| **Composition 2** | 19.8 | 9.3 | -14.1 | 17.4 | 4.7 | -7.8 | **8.2** |
| **Composition 3** | 20.2 | 8.9 | -13.6 | 18.6 | 6.5 | -11 | **3.9** |

Les résultats montrent une sélectivité plus faible pour la composition 2 de l'invention par rapport à la composition 3 comparative.

## Revendications

1. Composition de coloration des fibres kératiniques comprenant, dans un milieu approprié pour la teinture des fibres kératiniques :

   • Une ou plusieurs bases d'oxydation aminopyrazolopyridine de formule (I) suivante :

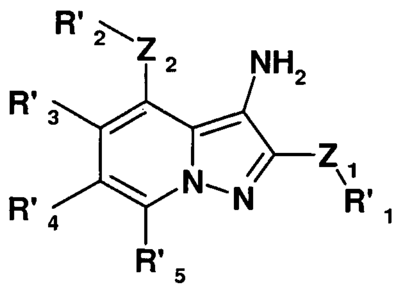

(I)

X

dans laquelle

• $Z_1$ et $Z_2$ représentent indépendamment

- une liaison covalente simple,
- un radical divalent choisi parmi
- un radical $-O(CH_2)_p-$, p désignant un nombre entier allant de 0 à 6
- un radical- $NR'_6$ $(CH_2)_q$ $(C_6H_4)_t-$, q désignant un nombre entier allant de 0 à 6 et t désignant 0 ou 1. $R'_6$ représente un atome d'hydrogène, un radical alkyle en $C_1$- $C_6$ éventuellement substitué par un ou plusieurs groupements hydroxy.

• $Z_1$ peut aussi représenter un radical divalent -S-, -SO-, $-SO_2-$ lorsque $R'_1$ est un radical méthyle,

• $R'_1$ et $R'_2$ représentent indépendamment :

- un hydrogène
  - un radical alkyle en $C_1$-$C_{10}$, éventuellement substitué et éventuellement interrompu par un hétéroatome ou un groupement choisi parmi O, N, Si, S, SO, $SO_2$,
  - un halogène,
  - un radical $SO_3H$,
  - un cycle de 5 à 8 chaînons, substitué ou non, saturé, insaturé ou aromatique, renfermant éventuellement un ou plusieurs hétéroatomes ou groupements choisis parmi N, O, S, $SO_2$, -CO-, le cycle pouvant être cationique et/ou substitué par un radical cationique,
  - un groupement- $N^+R_{17}R_{18}R_{19}$, $R_{17}$, $R_{18}$ et $R_{19}$ étant des alkyls linéaires ou ramifiés en $C_1$- $C_5$ éventuellement substituées par un ou plusieurs groupements hydroxy.

lorsque $Z_1$ respectivement $Z_2$ représente une liaison covalente, alors $R'_1$ respectivement $R'_2$ peut aussi représenter un radical :
- alkylcarbonyle en $C_1$-$C_6$, éventuellement substitué
- -O-CO-R, -CO-O-R, NR-CO-R' ou -CO-NRR' dans lesquels R et R' représentent indépendamment un atome d'hydrogène ou un radical alkyle en $C_1$-$C_6$ éventuellement substitué,

• $R'_3$, $R'_4$ et $R'_5$, identiques ou différents, représentent

- un atome d'hydrogène,
- un radical hydroxyle,
- un radical alcoxy en $C_1$-$C_6$,
- un radical alkylthio en $C_1$-$C_6$,
- un radical amino,
- un radical monoalkylamino,
- un radical dialkylamino en $C_1$-$C_6$ dans lequel les radicaux alkyles peuvent former avec l'atome d'azote auquel ils sont rattachés un hétérocycle de 5 à 8 chaînons, saturé, insaturé, aromatique ou non, pouvant renfermer un ou plusieurs hétéroatomes ou groupements choisis parmi N, O, S, $SO_2$, CO, l'hétérocycle pouvant être cationique, et/ou substitué par un radical cationique,
- un radical alkylcarbonyle en $C_1$-$C_6$, éventuellement substitué,
- un radical -O-CO-R, -CO-O-R, NR-CO-R' ou -CO-NRR' avec R et R' tels que définis précédemment,
- un halogène,
- un radical $-NHSO_3H$,
- un radical alkyle en $C_1$-$C_4$ éventuellement substitué,
- un cycle carboné saturé, insaturé ou aromatique, éventuellement substitué.
- $R'_3$, $R'_4$ et $R'_5$, peuvent former deux à deux un cycle saturé ou non,

• X représente un ion ou groupe d'ions permettant d'assurer l'électronégativité du dérivé de formule (I),

avec la condition qu'au moins un des groupements $R'_1$ et $R'_2$ représente un radical cationique.

• un ou plusieurs coupleurs,

• un ou plusieurs polyols en $C_4$-$C_{30}$ comportant une chaîne hydrocarbonée, linéaire, ramifiée ou cyclique, saturée ou insaturée, portant au moins deux fonctions hydroxyle, ladite chaîne et ses ramifications étant éventuellement

substitués par un

ou plusieurs autres substituants choisis parmi les groupements carboxy, amino, halogène, aryle en $C_6$-$C_{30}$ ; un ou plusieurs polyéthylène glycol à 4, 6 ou 7 motifs d'éthylène et le dipropylène glycol.

**2.** Composition selon la revendication 1 **caractérisé en ce que**, dans la formule (I), $Z_1$ et/ou $Z_2$ représente une liaison covalente, un radical- $NR'_6$ $(CH_2)_q$ ou un radical- O $(CH_2)_p$- et $R'_1$ et/ou $R'_2$ est un radical cationique..

**3.** Composition selon la revendication 2 dans laquelle $R'_1$ ou $R'_2$ désignent les imidazoles substitués par un radical ammonium quaternaire ou les imidazoliums, les pipérazines substitués par un radical ammonium quaternaire ou les pipéraziniums, les pyrrolidines substitués par un radical ammonium quaternaire ou les pyrrolidiniums, les diazépanes substitués par un radical ammonium quaternaire ou les diazépaniums..

**4.** Composition selon la revendication 1 à 2 dans laquelle $R'_1$ et $R'_2$ représentent indépendamment un atome d'hydrogène, ou un groupement trialkylammonium, tri (hydroxyalkyl) ammonium, hydroxyalkyl- dialkyl- ammonium ou di (hydroxyalkyl) alkylammonium.

**5.** Composition selon l'une quelconque des revendications précédentes dans laquelle les radicaux $R'_3$, $R'_4$ et $R'_5$ indépendamment représentent un atome d'hydrogène, un radical alkyle en $C_1$-$C_4$ pouvant être substitué.

**6.** Composition selon l'une quelconque des revendications 1 à 5 dans laquelle le composé de formule (I) correspond à la formule (I') suivante :

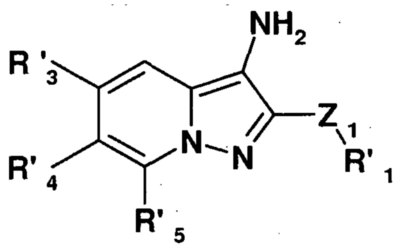

(I')

dans laquelle $Z_1$, $R'_1$, $R'_3$, $R'_4$ et $R'_5$ sont tels que définis à l'une quelconque des revendications 1 à 5.

**7.** Composition selon la revendication 6 dans laquelle $Z_1$ représente une liaison covalente, un radical- $NR'_6$ $(CH_2)_q$- ou un radical- O $(CH_2)_p$- et $R'_1$ est un radical cationique.

**8.** Composition selon l'une quelconque des revendications 1 à 7 dans laquelle le composé de formule (I) est choisi parmi :

| |
|---|
| 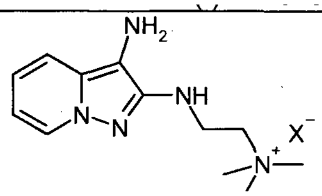<br>Sel de [2-(3-Amino-pyrazolo[1,5-a]pyridin-2-ylamino)-ethyl]-trimethyl-ammonium |
| 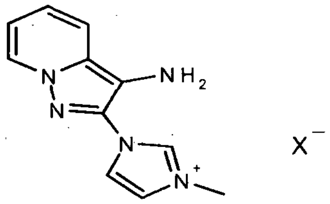<br>Sel de 3-(3-Amino-pyrazolo[1,5-a]pyridin-2-yl)-1-methyl-3H-imidazol-1-ium |
| 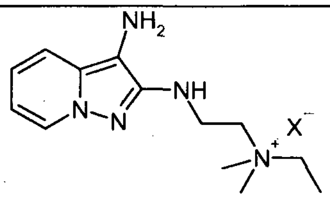<br>Sel de [2-(3-Amino-pyrazolo[1,5-a]pyridin-2-ylamino)-ethyl]-ethyl-dimethyl-ammonium |
| 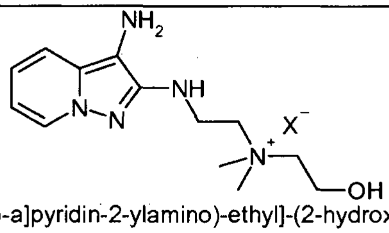<br>Sel de [2-(3-Amino-pyrazolo[1,5-a]pyridin-2-ylamino)-ethyl]-(2-hydroxy-ethyl)-dimethyl-ammonium |
| 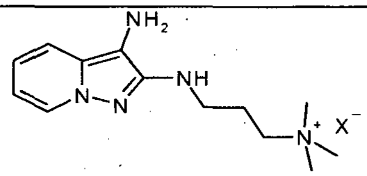<br>Sel de [3-(3-Amino-pyrazolo[1,5-a]pyridin-2-ylamino)-propyl]-trimethyl-ammonium |
| 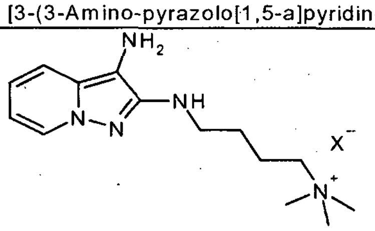<br>Sel de [4-(3-Amino-pyrazolo[1,5-a]pyridin-2-ylamino)-butyl]-trimethyl-ammonium |

| |
|---|
| 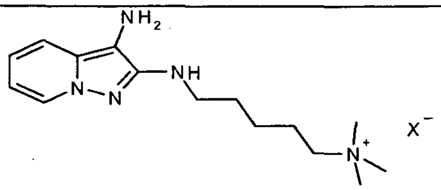<br>Sel de [5-(3-Amino-pyrazolo[1,5-a]pyridin-2-ylamino)-pentyl]-trimethyl-ammonium |
| 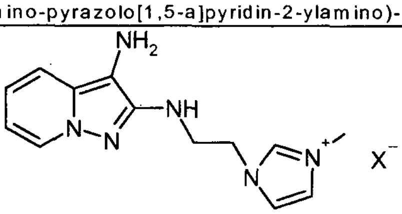<br>Sel de 3-[2-(3-Amino-pyrazolo[1,5-a]pyridin-2-ylamino)-ethyl]-1-methyl-3H-imidazol-1-ium |
| 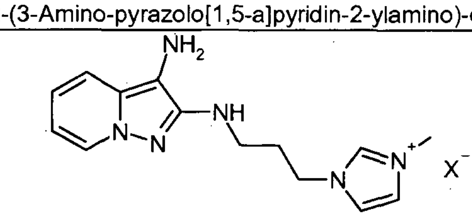<br>Sel de 3-[3-(3-Amino-pyrazolo[1,5-a]pyridin-2-ylamino)-propyl]-1-methyl-3H-imidazol-1-ium |
| 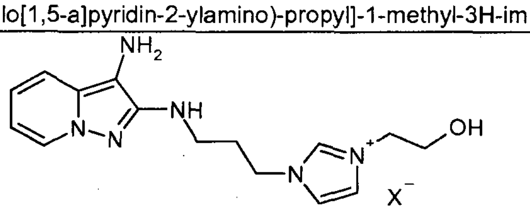<br>Sel de 3-[3-(3-Amino-pyrazolo[1,5-a]pyridin-2-ylamino)-propyl]-1-(2-hydroxy-ethyl)-3H-imidazol-1-ium |
| 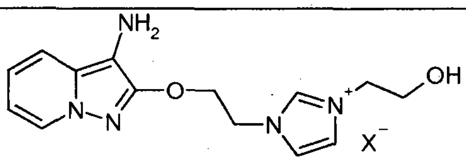<br>Sel de 3-[2-(3-Amino-pyrazolo[1,5-a]pyridin-2-yloxy)-ethyl]-1-(2-hydroxy-ethyl)-3H-imidazol-1-ium |
| 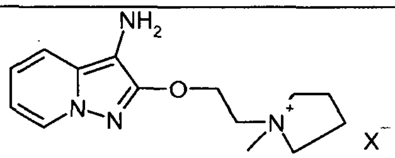<br>Sel de 1-{2-[(3-aminopyrazolo[1,5-a]pyridin-2-yl)oxy]ethyl}-1-methylpyrrolidinium |
| 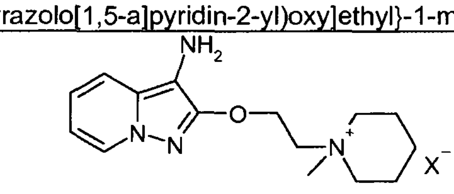<br>Sel de 1-{2-[(3-aminopyrazolo[1,5-a]pyridin-2-yl)oxy]ethyl}-1-methylpiperidinium |
| 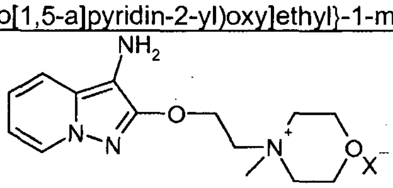 |

| |
|---|
| Sel de 4-{2-[(3-aminopyrazolo[1,5-a]pyridin-2-yl)oxy]ethyl}-4-methylmorpholin-4-ium |
| 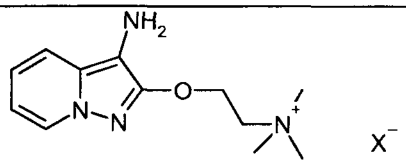 Sel de {2-[(3-aminopyrazolo[1,5-a]pyridin-2-yl)oxy]ethyl}- trimethyl-ammonium |
| 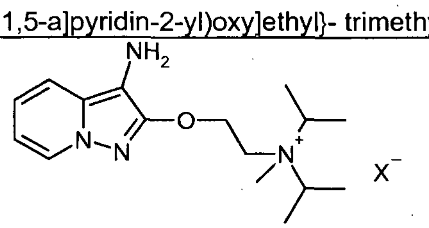 Sel de {2-[(3-aminopyrazolo[1,5-a]pyridin-2-yl)oxy]ethyl}-diisopropyl-methyl-ammonium |
| 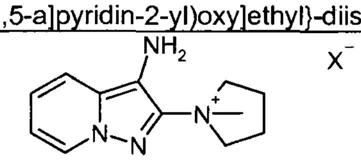 Sel de 1-(3-aminopyrazolo[1,5-a]pyridin-2-yl)-1-methylpyrrolidinium |
| 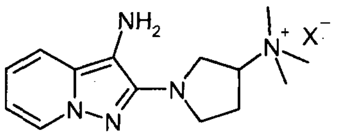 Sel de [1-(3-Amino-pyrazolo[1,5-a]pyridin-2-yl)-pyrrolidin-3-yl]-trimethyl-ammonium |
| 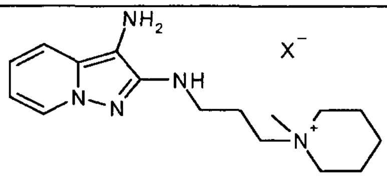 Sel de 1-[3-(3-Amino-pyrazolo[1,5-a]pyridin-2-ylamino)-propyl]-1-methylpiperidinium |
| 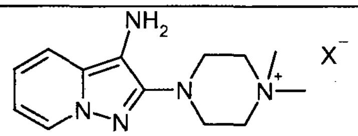 Sel de 4-(3-Amino-pyrazolo[1,5-a]pyridin-2-yl)-1,1-dimethyl-piperazin-1-ium |
| 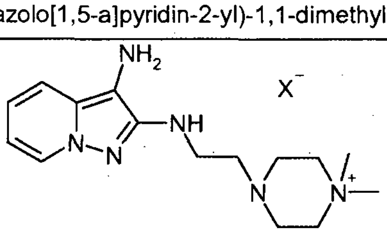 Sel de 4-[2-(3-Amino-pyrazolo[1,5-a]pyridin-2-ylamino)-ethyl]-1,1-dimethyl-piperazin-1-ium |

| |
|---|
| 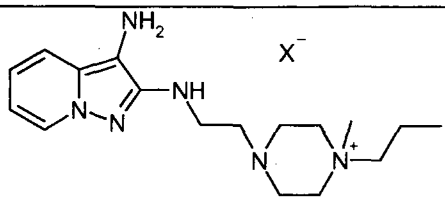<br>Sel de 4-[2-(3-Amino-pyrazolo[1,5-a]pyridin-2-ylamino)-ethyl]-1-methyl-1-propyl-piperazin-1-ium |
| 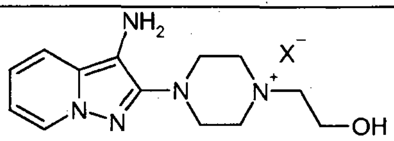<br>Sel de 4-(3-Amino-pyrazolo[1,5-a]pyridin-2-yl)-1-(2-hydroxy-ethyl)-piperazin-1-ium |
| 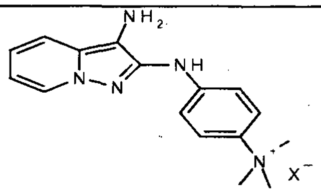<br>Sel de [4-(3-Amino-pyrazolo[1,5-a]pyridin-2-ylamino)-phenyl]-trimethyl-ammonium |
| 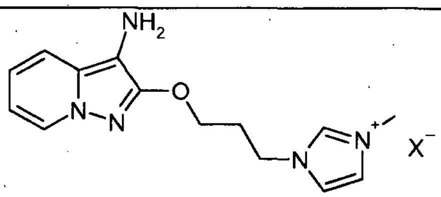<br>Sel de 3-[3-(3-Amino-pyrazolo[1,5-a]pyridin-2-yloxy)-propyl]-1-methyl-3H-imidazol-1-ium |
| 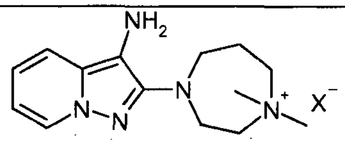<br>Sel de 4-(3-Amino-pyrazolo[1,5-a]pyridin-2-yl)-1,1-dimethyl-[1,4]diazepan-1-ium |
| 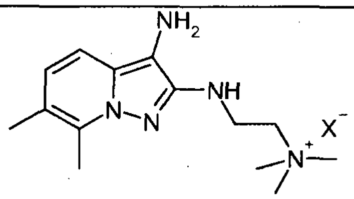<br>Sel de [2-(3-Amino-6,7-dimethyl-pyrazolo[1,5-a]pyridin-2-ylamino)-ethyl]-trimethyl-ammonium |
| 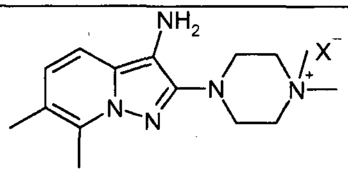<br>Sel de 4-(3-Amino-6,7-dimethyl-pyrazolo[1,5-a]pyridin-2-yl)-1,1-dimethyl-piperazin-1-ium |
| 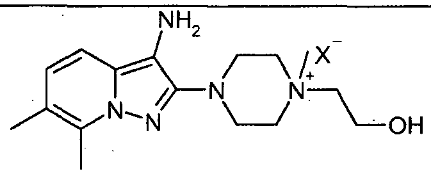<br>Sel de 4-(3-Amino-6,7-dimethyl-pyrazolo[1,5-a]pyridin-2-yl)-1-(2-hydroxy-ethyl)-1-methyl-piperazin-1-ium |

| |
|---|
| 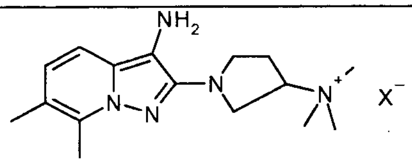 Sel de [1-(3-Amino-6,7-dimethyl-pyrazolo[1,5-a]pyridin-2-yl)-pyrrolidin-3-yl]-trimethyl-ammonium |
| 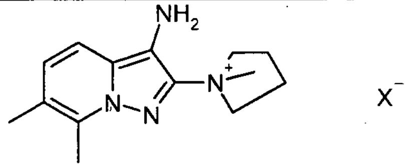 Sel de 1-(3-amino-6,7-dimethylpyrazolo[1,5-a]pyridin-2-yl)-1-methylpyrrolidinium |
| 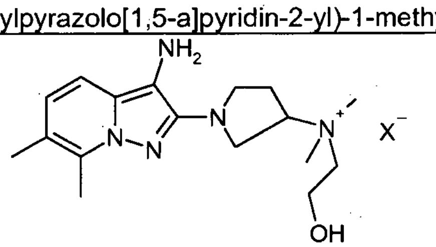 Sel de [1-(3-Amino-6,7-dimethyl-pyrazolo[1,5-a]pyridin-2-yl)-pyrrolidin-3-yl]-(2-hydroxy-ethyl)-dimethyl-ammonium |
| 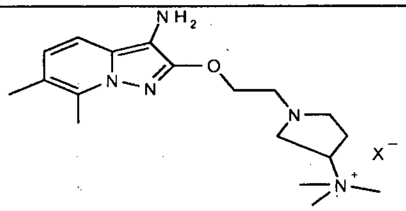 Sel de {1-[2-(3-Amino-6,7-dimethyl-pyrazolo[1,5-a]pyridin-2-yloxy)-ethyl]-pyrrolidin-3-yl}-trimethyl-ammonium |
| 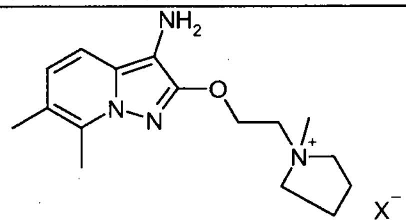 Sel de 1-{2-[(3-amino-6,7-dimethylpyrazolo[1,5-a]pyridin-2-yl)oxy]ethyl}-1-methyl pyrrolidinium |
| 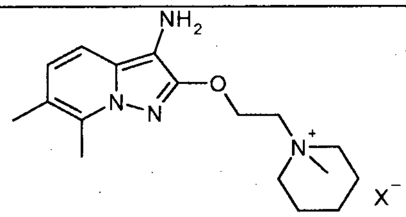 Sel de 1-{2-[(3-amino-6,7-dimethylpyrazolo[1,5-a]pyridin-2-yl)oxy]ethyl}-1-methylpiperidinium |

| |
|---|
| 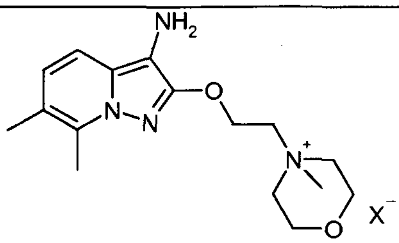<br>Sel de 4-{2-[(3-amino-6,7-dimethylpyrazolo[1,5-a]pyridin-2-yl)oxy]ethyl}-4-methylmorpholin-4-ium |
| 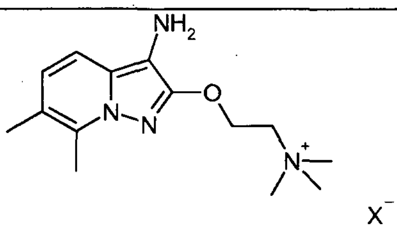<br>Sel de {2-[(3-amino-6,7-dimethylpyrazolo[1,5-a]pyridin-2-yl)oxy]ethyl}-trimethyl-ammonium |
| 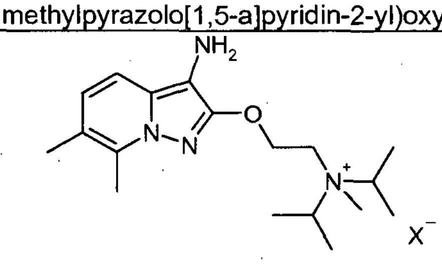<br>Sel de {2-[(3-amino-6,7-dimethylpyrazolo[1,5-a]pyridin-2-yl)oxy]ethyl}-diisopropyl-methyl-ammonium |
| 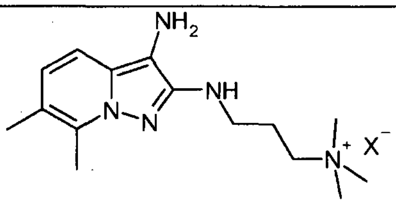<br>Sel de [3-(3-Amino-6,7-dimethyl-pyrazolo[1,5-a]pyridin-2-ylamino)-propyl]-trimethyl-ammonium |
| 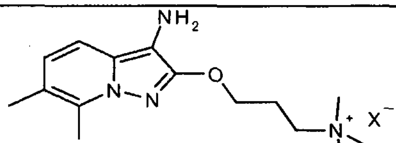<br>Sel de [3-(3-Amino-6,7-dimethyl-pyrazolo[1,5-a]pyridin-2-yloxy)-propyl]-trimethyl-ammonium |
| 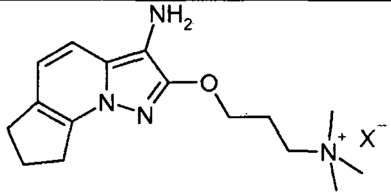<br>Sel de [3-(3-Amino-7,8-dihydro-6H-cyclopenta[e]pyrazolo[1,5-a]pyridin-2-yloxy)-propyl]-trimethyl-ammonium |

| |
|---|
| 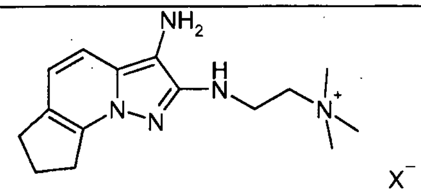<br>Sel de {2-[(3-amino-7,8-dihydro-6H-cyclopenta[e]pyrazolo[1,5-a]pyridin-2-yl)amino]-ethyl}-trimethyl-ammonium |
| 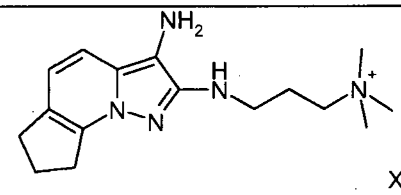<br>Sel de {3-[(3-amino-7,8-dihydro-6H-cyclopenta[e]pyrazolo[1,5-a]pyridin-2-yl)amino]-propyl}-trimethyl-ammonium |
| 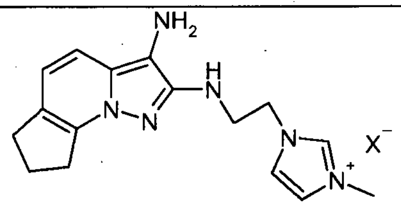<br>Sel de 1-{2-[(3-amino-7,8-dihydro-6H-cyclopenta[e]pyrazolo[1,5-a]pyridin-2-yl)amino]ethyl}-3-methyl-1H-imidazol-3-ium |
| 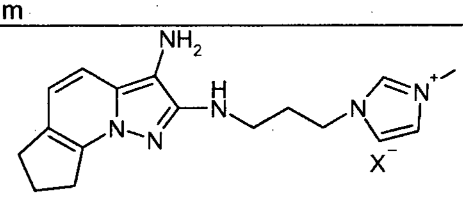<br>Sel de 1-{3-[(3-amino-7,8-dihydro-6H-cyclopenta[e]pyrazolo[1,5-a]pyridin-2-yl)amino]propyl}-3-methyl-1H-imidazol-3-ium |
| 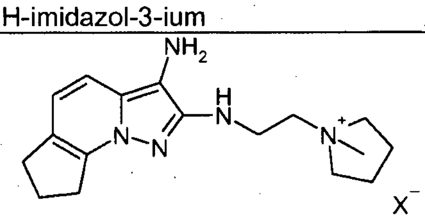<br>Sel de 1-{2-[(3-amino-7,8-dihydro-6H-cyclopenta[e]pyrazolo[1,5-a]pyridin-2-yl)amino]ethyl}-1-methylpyrrolidinium |
| 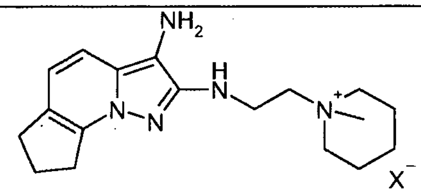<br>Sel de 1-{2-[(3-amino-7,8-dihydro-6H-cyclopenta[e]pyrazolo[1,5-a]pyridin-2-yl)amino]ethyl}-1-methylpiperidinium |

| |
|---|
| 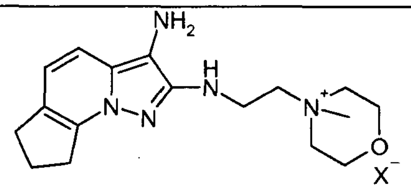<br>Sel de 4-{2-[(3-amino-7,8-dihydro-6H-cyclopenta[e]pyrazolo[1,5-a]pyridin-2-yl)amino]ethyl}-4-methylmorpholin-4-ium |
| 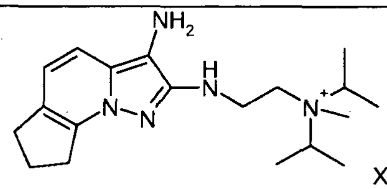<br>Sel de {2-[(3-amino-7,8-dihydro-6H-cyclopenta[e]pyrazolo[1,5-a]pyridin-2-yl)amino]ethyl}-diisopropyl-methyl-ammonium |
| 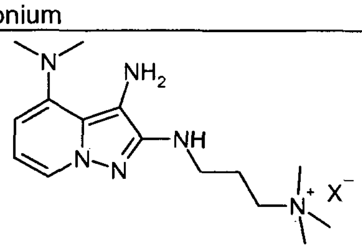<br>Sel de [3-(3-Amino-4-dimethylamino-pyrazolo[1,5-a]pyridin-2-ylamino)-propyl]-trimethyl-ammonium |
| 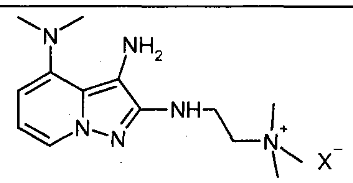<br>Sel de [2-(3-Amino-4-dimethylamino-pyrazolo[1,5-a]pyridin-2-ylamino)-ethyl]-trimethyl-ammonium |
| 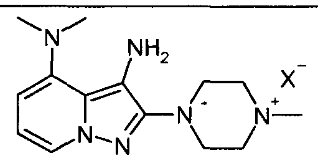<br>Sel de 4-(3-Amino-4-dimethylamino-pyrazolo[1,5-a]pyridin-2-yl)-1-methyl-piperazin-1-ium |
| 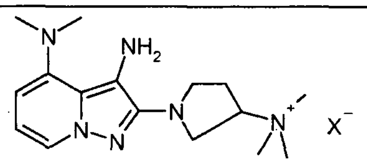<br>Sel de [1-(3-Amino-4-dimethylamino-pyrazolo[1,5-a]pyridin-2-yl)-pyrrolidin-3-yl]-trimethyl-ammonium |
| 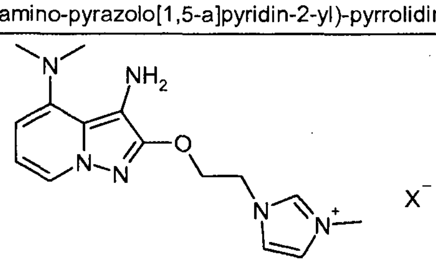<br>Sel de 3-[2-(3-Amino-4-dimethylamino-pyrazolo[1,5-a]pyridin-2-yloxy)-ethyl]-1-methyl-3H-imidazol-1-ium |

| |
|---|
| 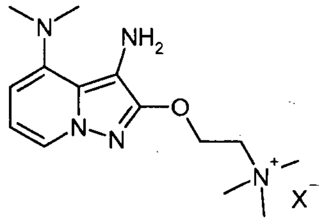 Sel de [2-(3-Amino-4-dimethylamino-pyrazolo[1,5-a]pyridin-2-yloxy)-ethyl]-trimethyl-ammonium |
| 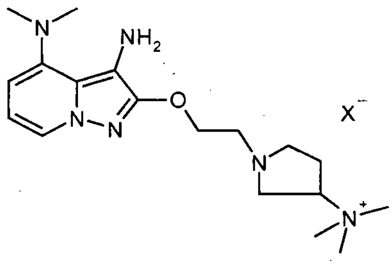 Sel de {1-[2-(3-Amino-4-dimethylamino-pyrazolo[1,5-a]pyridin-2-yloxy)-ethyl]-pyrrolidin-3-yl}-trimethyl-ammonium |
| 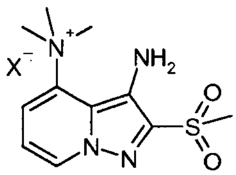 Sel de (3-Amino-2-methanesulfonyl-pyrazolo[1,5-a]pyridin-4-yl)-trimethyl-ammonium |
| 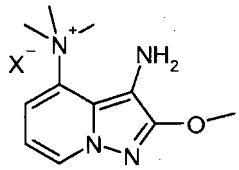 Sel de (3-Amino-2-methoxy-pyrazolo[1,5-a]pyridin-4-yl)-trimethyl-ammonium |

**9.** Composition selon l'une quelconque des revendications précédentes, **caractérisée en ce que** la teneur en composé de formule (I) est comprise entre 0,001 à 10 % en poids environ du poids total de la composition tinctoriale.

**10.** Composition selon l'une quelconque des revendications précédentes, **caractérisée en ce que** le ou les coupleurs sont choisis parmi les métaphénylènediamines, les méta-aminophénols, les métadiphénols, les coupleurs naphtaléniques, les coupleurs hétérocycliques et leurs sels d'addition.

**11.** Composition selon l'une quelconque des revendications précédentes dans laquelle le ou les polyols sont en $C_4$-$C_{15}$.

**12.** Composition selon la revendication 11 dans laquelle le ou les polyols sont choisis parmi l'hexylèneglycol ; le néopentylglycol ; l'isoprèneglycol.

**13.** Composition selon l'une quelconque des revendications précédentes dans laquelle le ou les polyols sont présents dans des proportions comprises entre 0,1 et 40 % en poids du poids total de la composition tinctoriale.

**14.** Procédé de teinture des fibres kératiniques, **caractérisé en ce qu'**une composition telle que définie dans l'une quelconque des revendications 1 à 13 est appliquée sur les fibres kératiniques en présence d'un agent oxydant pendant un temps suffisant pour développer la coloration désirée.

**15.** Procédé selon la revendication 14 dans lequel l'agent oxydant est choisi parmi le peroxyde d'hydrogène, le peroxyde d'urée, les bromates de métaux alcalins, les persels, les peracides et les enzymes oxydases.

**16.** Dispositif à plusieurs compartiments dans lequel un premier compartiment contient une composition tinctoriale telle

que définie dans l'une quelconque des revendications 1 à 13 et un deuxième compartiment contient un agent oxydant.

## Patentansprüche

1. Zusammensetzung zum Färben von Keratinfasern, umfassend in einem für das Färben von Keratinfasern geeigneten Medium:

• eine oder mehrere Aminopyrazolopyridin-Oxidationsbasen der folgenden Formel (I):

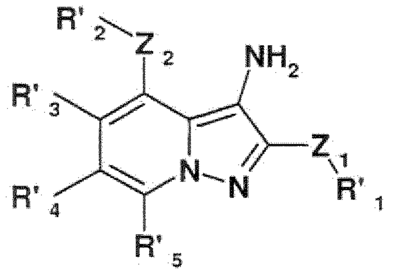

(I)

X

worin
• $Z_1$ und $Z_2$ unabhängig voneinander für

- eine kovalente Einfachbindung,
- einen zweiwertigen Rest, ausgewählt aus
- einem- O $(CH_2)_p$- Rest, wobei p für eine ganze Zahl im Bereich von 0 bis 6 steht,
- einem- NR'$_6$ $(CH_2)_q$ $(C_6H_4)_t$- Rest, wobei q für eine ganze Zahl im Bereich von 0 bis 6 steht und t für 0 oder 1 steht und R'$_6$ für ein Wasserstoffatom oder einen $C_1$- $C_6$- Alkylrest, der gegebenenfalls durch eine oder mehrere Hydroxygruppen substituiert ist, steht,

stehen,
• $Z_1$ auch für einen zweiwertigen -S-, -SO- oder -$SO_2$-Rest stehen kann, wenn R'$_1$ für einen Methylrest steht,
• R'$_1$ und R'$_2$ unabhängig voneinander für:

- einen Wasserstoff,
- einen $C_1$- $C_{10}$- Alkylrest, der gegebenenfalls substituiert und gegebenenfalls durch ein Heteroatom oder eine Gruppierung, ausgewählt aus O, N, Si, S, SO und $SO_2$, unterbrochen ist,
- ein Halogen,
- einen $SO_3H$-Rest,
- einen gesättigten, ungesättigten oder aromatischen, substituierten oder unsubstituierten 5- bis 8-gliedrigen Ring, der gegebenenfalls ein oder mehrere Heteroatome oder Gruppen, ausgewählt aus N, O, S, $SO_2$ und -CO-, enthält und kationisch sein und/oder durch einen kationischen Rest substituiert sein kann,
- eine- $N^+R_{17}R_{18}R_{19}$- Gruppe, wobei $R_{17}$, $R_{18}$ und $R_{19}$ für lineare oder verzweigte $C_1$- $C_5$- Alkylgruppen, die gegebenenfalls durch eine oder mehrere Hydroxygruppen substituiert sind, stehen,

stehen,
wenn $Z_1$ bzw. $Z_2$ für eine kovalente Bindung steht, R'$_1$ bzw. R'$_2$ auch für:

- einen gegebenenfalls substituierten $C_1$- $C_6$- Alkylcarbonylrest,
- einen- O- CO- R-, - CO- O- R-, NR- CO- R'- oder- CO- NRR'- Rest, worin R und R' unabhängig voneinander für ein Wasserstoffatom oder einen gegebenenfalls substituierten $C_1$- $C_6$- Alkylrest stehen,

stehen können,
• R'$_3$, R'$_4$ und R'$_5$ gleich oder verschieden sind und für

- ein Wasserstoffatom,
- einen Hydroxylrest,

- einen $C_1$- $C_6$- Alkoxyrest,
- einen $C_1$- $C_6$- Alkylthiorest,
- einen Aminorest,
- einen Monoalkylaminorest,
- einen $C_1$- $C_6$- Dialkylaminorest, worin die Alkylreste mit dem Stickstoffatom, an das sie gebunden sind, einen gesättigten, ungesättigten, aromatischen oder nichtaromatischen 5- bis 8- gliedrigen Heterocyclus, der gegebenenfalls ein oder mehrere Heteroatome oder Gruppen, ausgewählt aus N, O, S, $SO_2$ und CO, enthält und kationisch sein und/ oder durch einen kationischen Rest substituiert sein kann, bilden können,
- einen gegebenenfalls substituierten $C_1$- $C_6$- Alkylcarbonylrest,
- einen- O- CO- R-, - CO- O- R-, NR- CO- R'- oder- CO- NRR'- Rest, wobei R und R' wie oben definiert sind,
- ein Halogen,
- einen- $NHSO_3H$- Rest,
- einen gegebenenfalls substituierten $C_1$- $C_4$- Alkylrest,
- einen gesättigten, ungesättigten oder aromatischen, gegebenenfalls substituierten Kohlenstoffring stehen,
- wobei $R'_3$, $R'_4$ und $R'_5$ paarweise einen gesättigten oder ungesättigten Ring bilden können,

• X für ein Ion oder eine Gruppe von Ionen steht, so dass die Elektronegativität der Verbindung der Formel (I) gewährleistet ist,
mit der Maßgabe, dass mindestens eine der Gruppen $R'_1$ und $R'_2$ für einen kationischen Rest steht,
• einen oder mehrere Kuppler,
• ein oder mehrere $C_4$- $C_{30}$- Polyole mit einer gesättigten oder ungesättigten, linearen, verzweigten oder cyclischen Kohlenwasserstoffkette mit mindestens zwei Hydroxylfunktionen, wobei die Kette und ihre Verzweigungen gegebenenfalls durch einen oder mehrere andere Substituenten, die aus Carboxy-, Amino-, Halogen- und $C_6$- $C_{30}$- Arylgruppen ausgewählt sind, substituiert sind; ein oder mehrere Polyethylenglykole mit 4, 6 oder 7 Ethylen- Einheiten und Dipropylenglykol.

**2.** Zusammensetzung nach Anspruch 1, **dadurch gekennzeichnet, dass** in der Formel (I) $Z_1$ und/ oder $Z_2$ für eine kovalente Bindung, einen- $NR'_6$ $(CH_2)_q$- Rest oder einen- O $(CH_2)_p$- Rest stehen und $R'_1$ und/ oder $R'_2$ für einen kationischen Rest stehen.

**3.** Zusammensetzung nach Anspruch 2, worin $R'_1$ oder $R'_2$ für Imidazolgruppen, die durch einen quaternären Ammoniumrest substituiert sind, oder Imidazoliumgruppen, Piperazingruppen, die durch einen quaternären Ammoniumrest substituiert sind, oder Piperaziniumgruppen, Pyrrolidingruppen, die durch einen quaternären Ammoniumrest substituiert sind, oder Pyrrolidiniumgruppen oder Diazepangruppen, die durch einen quaternären Ammoniumrest substituiert sind, oder Diazepaniumgruppen stehen.

**4.** Zusammensetzung nach Anspruch 1 bis 2, worin $R'_1$ und $R'_2$ unabhängig voneinander für ein Wasserstoffatom oder eine Trialkylammonium-, Tri (hydroxyalkyl) ammonium-, (Hydroxyalkyl) dialkylammonium- oder Di (hydroxyalkyl) alkylammoniumgruppe stehen.

**5.** Zusammensetzung nach einem der vorhergehenden Ansprüche, worin die Reste $R'_3$, $R'_4$ und $R'_5$ unabhängig voneinander für ein Wasserstoffatom oder einen gegebenenfalls substituierten $C_1$- $C_4$- Alkylrest stehen.

**6.** Zusammensetzung nach einem der Ansprüche 1 bis 5, worin die Verbindung der Formel (I) der folgenden Formel (I') entspricht:

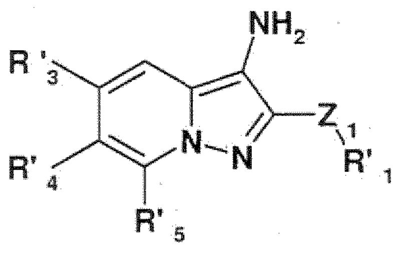

(I')

worin $Z_1$, $R'_1$, $R'_3$, $R'_4$ und $R'_5$ wie in einem der Ansprüche 1 bis 5 definiert sind.

**7.** Zusammensetzung nach Anspruch 6, worin $Z_1$ für eine kovalente Bindung, einen- $NR'_6$ $(CH_2)_q$- Rest oder einen- O $(CH_2)_p$- Rest steht und $R'_1$ für einen kationischen Rest steht.

**8.** Zusammensetzung nach einem der Ansprüche 1 bis 7, worin die Verbindung der Formel (I) ausgewählt ist aus:

| |
|---|
| 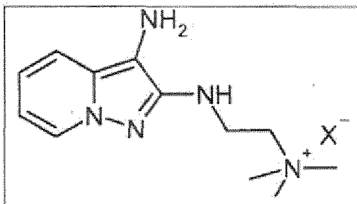 [2-(3-Aminopyrazolo[1,5-a]pyridin-2-ylamino)ethyl]trimethylammoniumsalz |
| 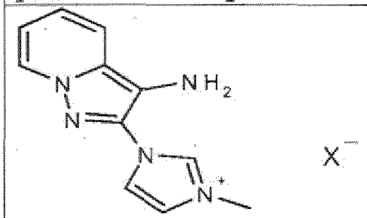 3-(3-Aminopyrazolo[1,5-a]pyridin-2-yl)-1-methyl-3H-imidazol-1-iumsalz |
| 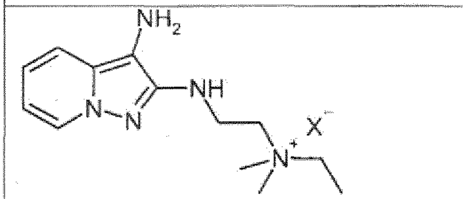 [2-(3-Aminopyrazolo[1,5-a]pyridin-2-ylamino)ethyl]ethyldimethylammoniumsalz |

| |
|---|
| 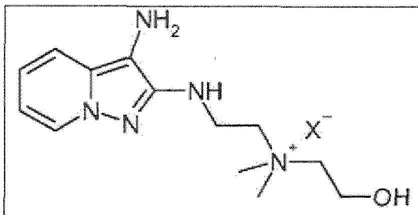 [2-(3-Aminopyrazolo[1,5-a]pyridin-2-ylamino)ethyl](2-hydroxyethyl)dimethylammoniumsalz |
| 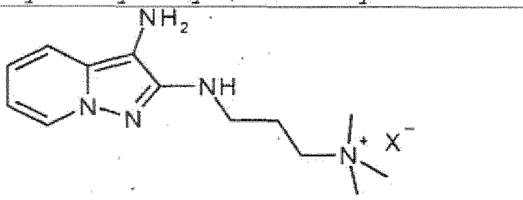 [3-(3-Aminopyrazolo[1,5-a]pyridin-2-ylamino)propyl]trimethylammoniumsalz |
| 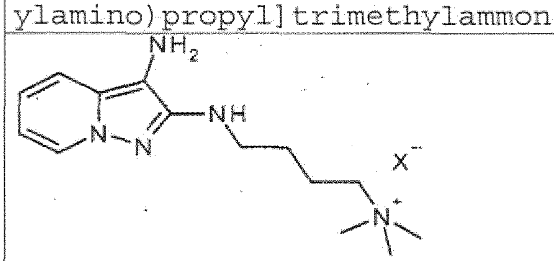 [4-(3-Aminopyrazolo[1,5-a]pyridin-2-ylamino)butyl]-trimethylammoniumsalz |
| 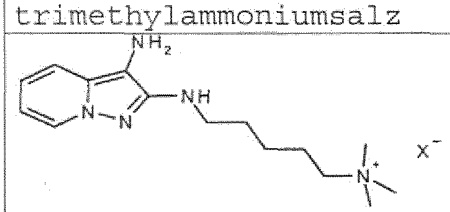 [5-(3-Aminopyrazolo[1,5-a]pyridin-2-ylamino)pentyl]trimethylammoniumsalz |
| 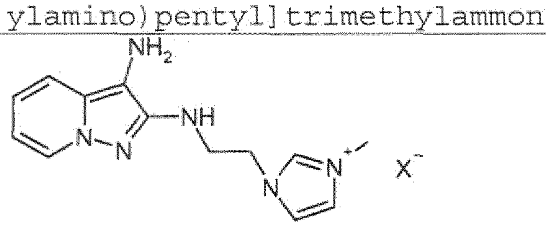 3-[2-(3-Aminopyrazolo[1,5-a]pyridin-2-ylamino)ethyl]-1-methyl-3H-imidazol-1-iumsalz |
| 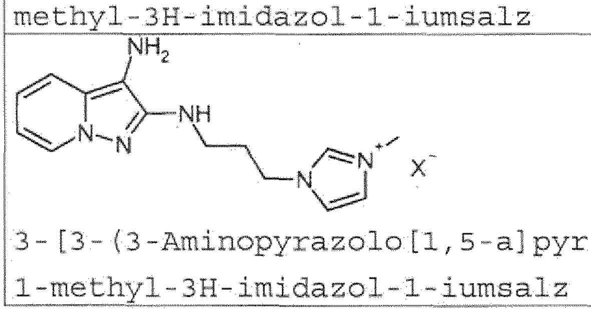 3-[3-(3-Aminopyrazolo[1,5-a]pyridin-2-ylamino)propyl]-1-methyl-3H-imidazol-1-iumsalz |

| |
|---|
| 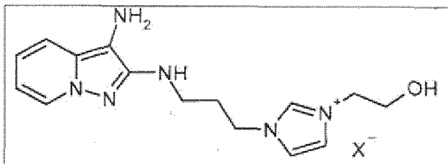 3-[3-(3-Aminopyrazolo[1,5-a]pyridin-2-ylamino)propyl]-1-(2-hydroxyethyl)-3H-imidazol-1-iumsalz |
| 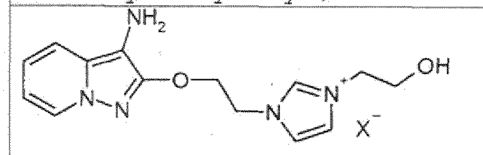 3-[2-(3-Aminopyrazolo[1,5-a]pyridin-2-yloxy)ethyl]-1-(2-hydroxyethyl)-3H-imidazol-1-iumsalz |
| 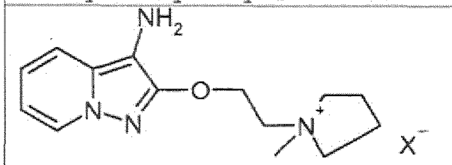 1-{2-[(3-Aminopyrazolo[1,5-a]pyridin-2-yl)oxy]ethyl}-1-methylpyrrolidiniumsalz |
| 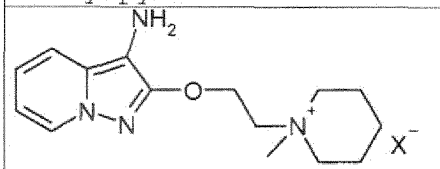 1-{2-[(3-Aminopyrazolo[1,5-a]pyridin-2-yl)oxy]ethyl}-1-methylpiperidiniumsalz |
| 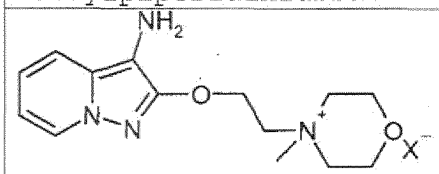 4-{2-[(3-Aminopyrazolo[1,5-a]pyridin-2-yl)oxy]ethyl}-4-methylmorpholin-4-iumsalz |
| 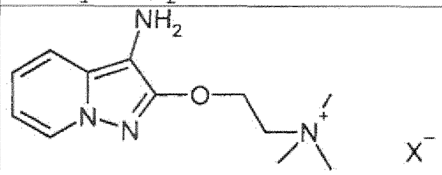 {2-[(3-Aminopyrazolo[1,5-a]pyridin-2-yl)oxy]ethyl}trimethylammoniumsalz |
| 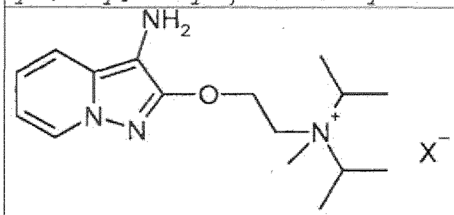 {2-[(3-Aminopyrazolo[1,5-a]pyridin-2- |

| |
|---|
| yl)oxy]ethyl}diisopropylmethylammoniumsalz |
| 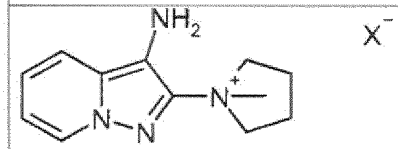 1-(3-Aminopyrazolo[1,5-a]pyridin-2-yl)-1-methylpyrrolidiniumsalz |
| 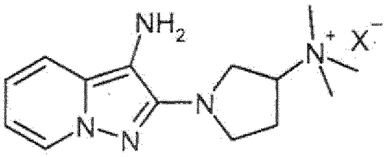 [1-(3-Aminopyrazolo[1,5-a]pyridin-2-yl)pyrrolidin-3-yl]trimethylammoniumsalz |
| 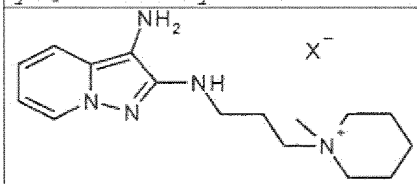 1-[3-(3-Aminopyrazolo[1,5-a]pyridin-2-ylamino)propyl]-1-methylpiperidiniumsalz |
| 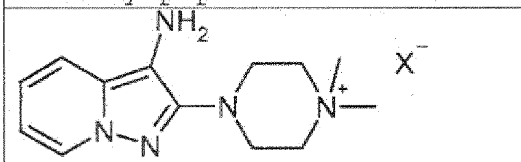 4-(3-Aminopyrazolo[1,5-a]pyridin-2-yl)-1,1-dimethylpiperazin-1-iumsalz |
| 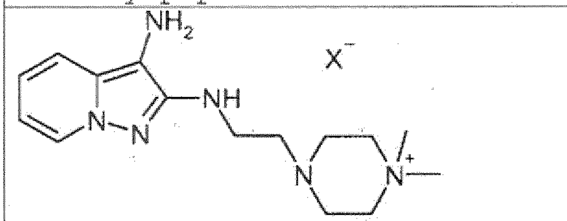 4-[2-(3-Aminopyrazolo[1,5-a]pyridin-2-ylamino)ethyl]-1,1-dimethylpiperazin-1-iumsalz |
| 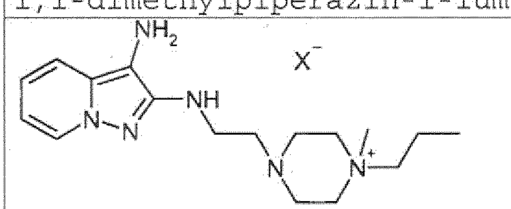 4-[2-(3-Aminopyrazolo[1,5-a]pyridin-2-ylamino)ethyl]-1-methyl-1-propylpiperazin-1-iumsalz |

| |
|---|
| 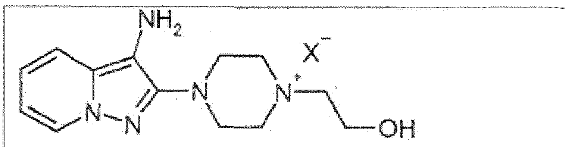<br>4-(3-Aminopyrazolo[1,5-a]pyridin-2-yl)-1-(2-hydroxyethyl)piperazin-1-iumsalz |
| 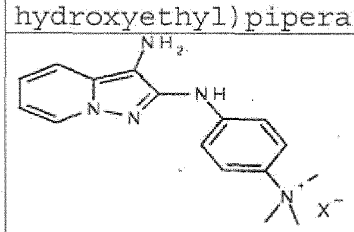<br>[4-(3-Aminopyrazolo[1,5-a]pyridin-2-ylamino)phenyl]trimethylammoniumsalz |
| 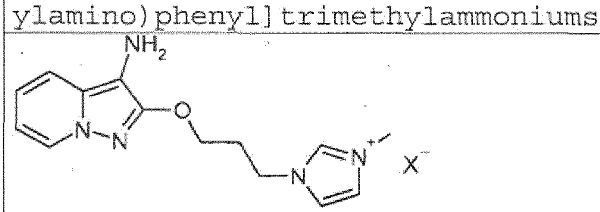<br>3-[3-(3-Aminopyrazolo[1,5-a]pyridin-2-yloxy)propyl]-1-methyl-3H-imidazol-1-iumsalz |
| 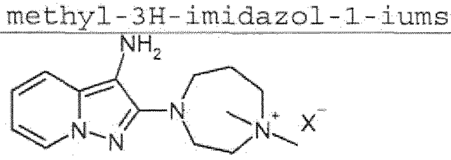<br>4-(3-Aminopyrazolo[1,5-a]pyridin-2-yl)-1,1-dimethyl-[1,4]diazepan-1-iumsalz |
| 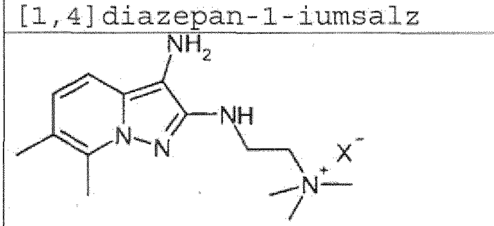<br>[2-(3-Amino-6,7-dimethylpyrazolo[1,5-a]pyridin-2-ylamino)ethyl]trimethylammoniumsalz |
| 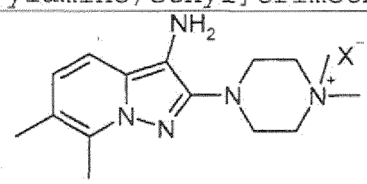<br>4-(3-Amino-6,7-dimethylpyrazolo[1,5-a]pyridin-2-yl)-1,1-dimethylpiperazin-1-iumsalz |
| 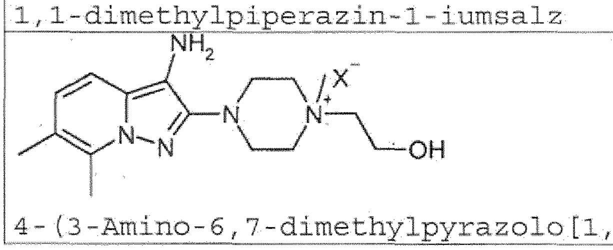<br>4-(3-Amino-6,7-dimethylpyrazolo[1,5-a]pyridin-2-yl)-1- |

| |
|---|
| (2-hydroxyethyl)-1-methylpiperazin-1-iumsalz |
| 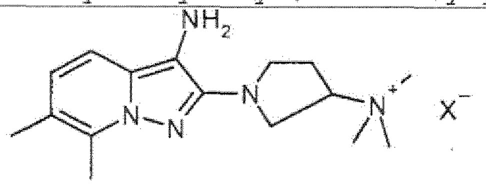 [1-(3-Amino-6,7-dimethylpyrazolo[1,5-a]pyridin-2-yl)pyrrolidin-3-yl]trimethylammoniumsalz |
| 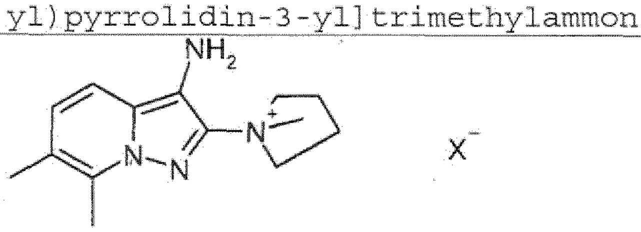 1-(3-Amino-6,7-dimethylpyrazolo[1,5-a]pyridin-2-yl)-1-methylpyrrolidiniumsalz |
| 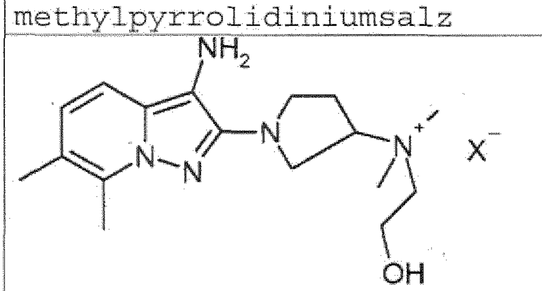 [1-(3-Amino-6,7-dimethylpyrazolo[1,5-a]pyridin-2-yl)pyrrolidin-3-yl](2-hydroxyethyl)dimethylammoniumsalz |
| 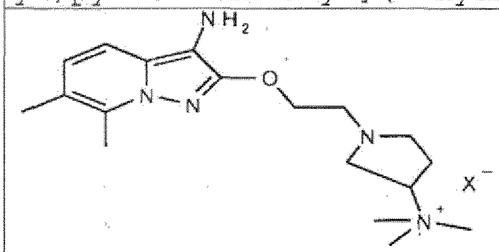 {1-[2-(3-Amino-6,7-dimethylpyrazolo[1,5-a]pyridin-2-yloxy)ethyl]pyrrolidin-3-yl}trimethylammoniumsalz |
| 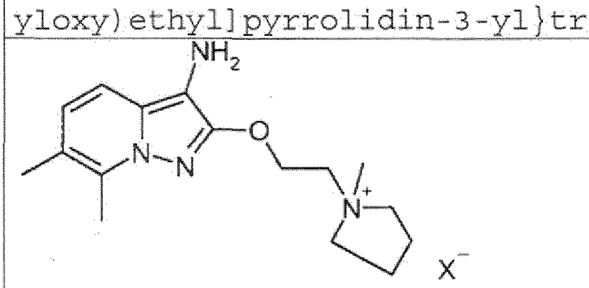 1-{2-[(3-Amino-6,7-dimethylpyrazolo[1,5-a]pyridin-2-yl)oxy]ethyl}-1-methylpyrrolidiniumsalz |

| |
|---|
| 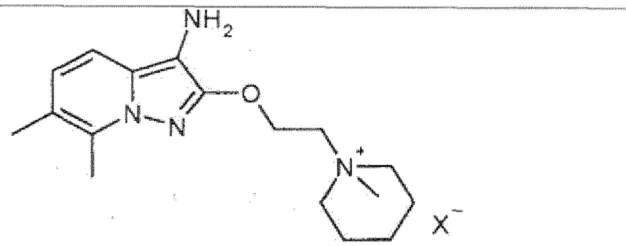<br>1-{2-[(3-Amino-6,7-dimethylpyrazolo[1,5-a]pyridin-2-yl)oxy]ethyl}-1-methylpiperidiniumsalz |
| 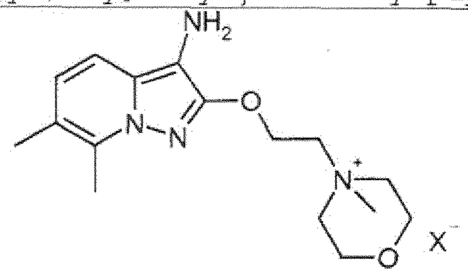<br>4-{2-[(3-Amino-6,7-dimethylpyrazolo[1,5-a]pyridin-2-yl)oxy]ethyl}-4-methylmorpholin-4-iumsalz |
| 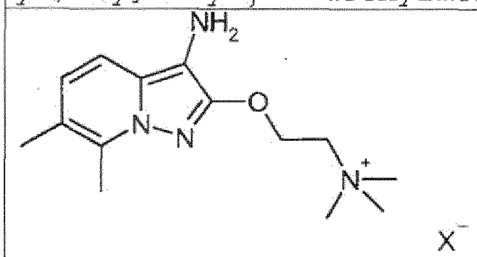<br>{2-[(3-Amino-6,7-dimethylpyrazolo[1,5-a]pyridin-2-yl)oxy]ethyl}trimethylammoniumsalz |
| 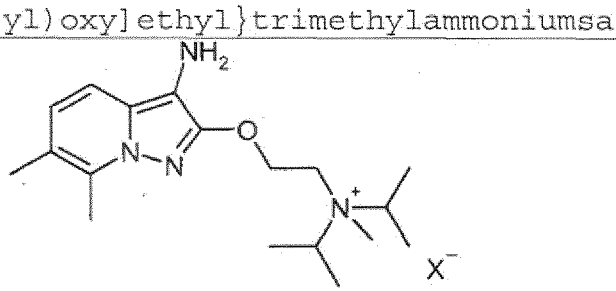<br>{2-[(3-Amino-6,7-dimethylpyrazolo[1,5-a]pyridin-2-yl)oxy]ethyl}diisopropylmethylammoniumsalz |
| 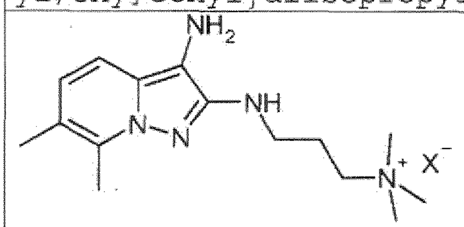<br>[3-(3-Amino-6,7-dimethylpyrazolo[1,5-a]pyridin-2-ylamino)propyl]trimethylammoniumsalz |

| |
|---|
| 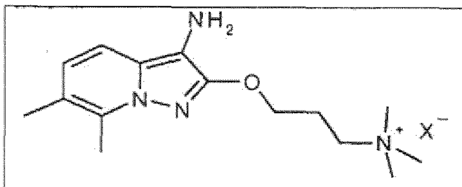<br>[3-(3-Amino-6,7-dimethylpyrazolo[1,5-a]pyridin-2-yloxy)propyl]trimethylammoniumsalz |
| 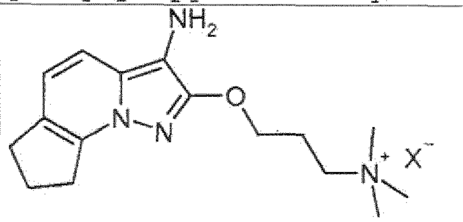<br>[3-(3-Amino-7,8-dihydro-6H-cyclopenta[e]pyrazolo[1,5-a]pyridin-2-yloxy)propyl]trimethylammoniumsalz |
| 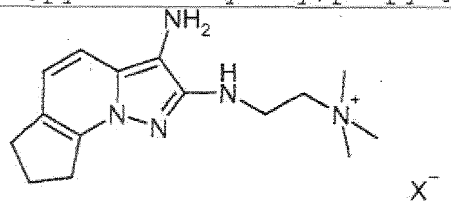<br>{2-[(3-Amino-7,8-dihydro-6H-cyclopenta[e]pyrazolo[1,5-a]pyridin-2-yl)amino]ethyl}trimethylammoniumsalz |
| 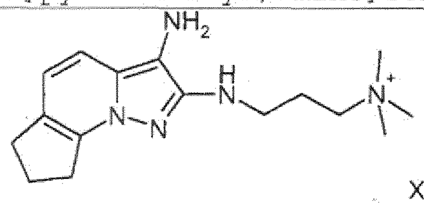<br>{3-[(3-Amino-7,8-dihydro-6H-cyclopenta[e]pyrazolo[1,5-a]pyridin-2-yl)amino]propyl}trimethylammoniumsalz |
| 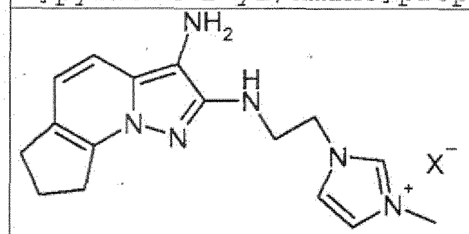<br>1-{2-[(3-Amino-7,8-dihydro-6H-cyclopenta[e]pyrazolo[1,5-a]pyridin-2-yl)amino]ethyl}-3-methyl-1H-imidazol-3-iumsalz |
| 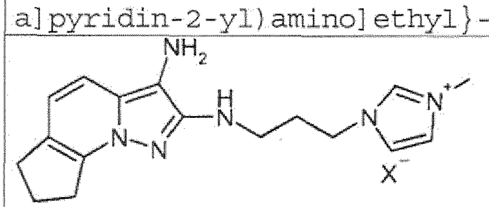<br>1-{3-[(3-Amino-7,8-dihydro-6H-cyclopenta[e]pyrazolo[1,5-a]pyridin-2-yl)amino]propyl}- |

| |
|---|
| 3-methyl-1H-imidazol-3-iumsalz |
| 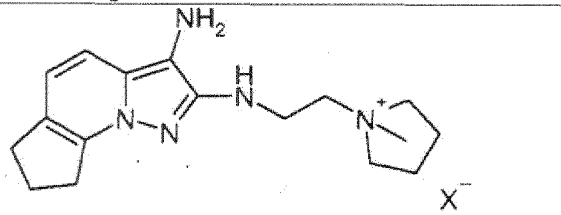 1-{2-[(3-Amino-7,8-dihydro-6H-cyclopenta[e]pyrazolo[1,5-a]pyridin-2-yl)amino]ethyl}-1-methylpyrrolidiniumsalz |
| 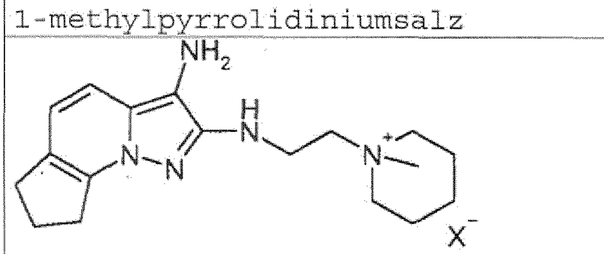 1-{2-[(3-Amino-7,8-dihydro-6H-cyclopenta[e]pyrazolo[1,5-a]pyridin-2-yl)amino]ethyl}-1-methylpiperidiniumsalz |
| 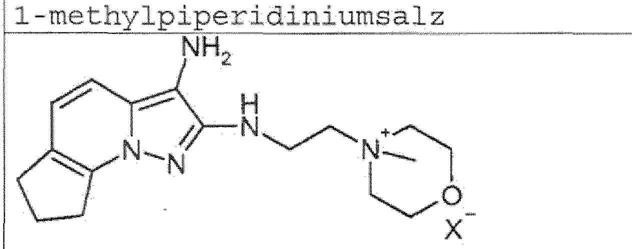 4-{2-[(3-Amino-7,8-dihydro-6H-cyclopenta[e]pyrazolo[1,5-a]pyridin-2-yl)amino]ethyl}-4-methylmorpholin-4-iumsalz |
| 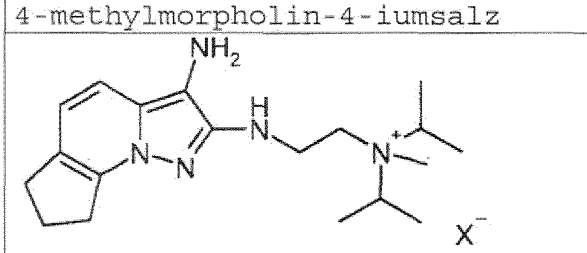 {2-[(3-Amino-7,8-dihydro-6H-cyclopenta[e]pyrazolo[1,5-a]pyridin-2-yl)amino]ethyl}diisopropylmethylammoniumsalz |
| 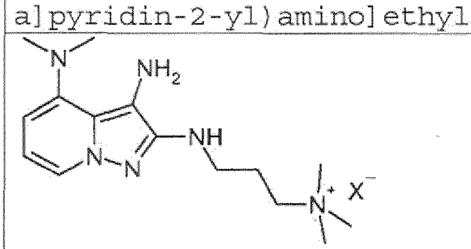 [3-(3-Amino-4-dimethylaminopyrazolo[1,5-a]pyridin-2-ylamino)propyl]trimethylammoniumsalz |

| |
|---|
| 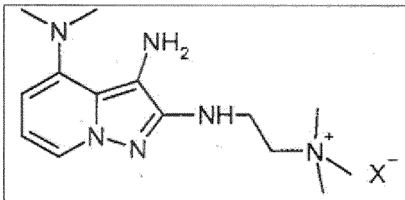 [2-(3-Amino-4-dimethylaminopyrazolo[1,5-a]pyridin-2-ylamino)ethyl]trimethylammoniumsalz |
| 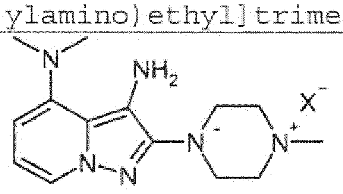 4-(3-Amino-4-dimethylaminopyrazolo[1,5-a]pyridin-2-yl)-1-methylpiperazin-1-iumsalz |
| 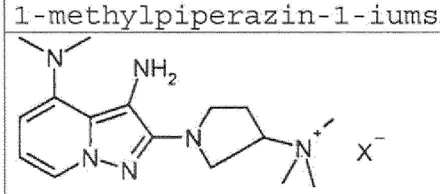 [1-(3-Amino-4-dimethylaminopyrazolo[1,5-a]pyridin-2-yl)pyrrolidin-3-yl]trimethylammoniumsalz |
| 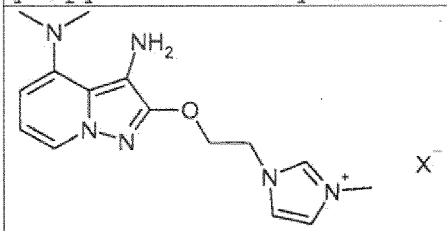 3-[2-(3-Amino-4-dimethylaminopyrazolo[1,5-a]pyridin-2-yloxy)ethyl]-1-methyl-3H-imidazol-1-iumsalz |
| 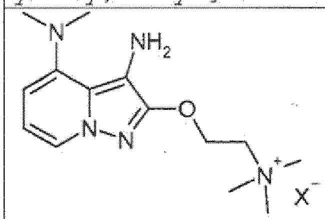 [2-(3-Amino-4-dimethylaminopyrazolo[1,5-a]pyridin-2-yloxy)ethyl]trimethylammoniumsalz |
| 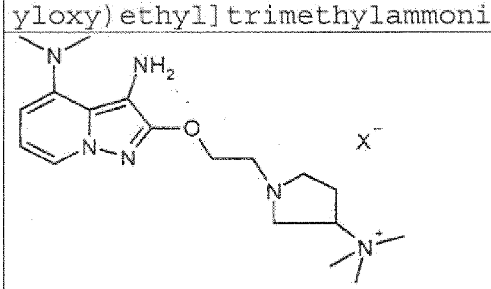 {1-[2-(3-Amino-4-dimethylaminopyrazolo[1,5-a]pyridin-2-yloxy)ethyl]pyrrolidin-3-yl}trimethylammoniumsalz |

| |
|---|
| 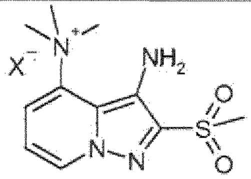 (3-Amino-2-methansulfonylpyrazolo[1,5-a]pyridin-4-yl)-trimethylammoniumsalz |
| 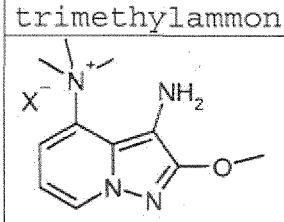 (3-Amino-2-methoxypyrazolo[1,5-a]pyridin-4-yl)trimethylammoniumsalz |

9. Zusammensetzung nach einem der vorhergehenden Ansprüche, **dadurch gekennzeichnet, dass** der Gehalt an der Verbindung der Formel (I) zwischen etwa 0,001 und 10 Gew.-%, bezogen auf das Gesamtgewicht der Färbezusammensetzung, liegt.

10. Zusammensetzung nach einem der vorhergehenden Ansprüche, **dadurch gekennzeichnet, dass** der Kuppler bzw. die Kuppler aus meta-Phenylendiaminen, meta-Aminophenolen, meta-Diphenolen, Naphthalin-Kupplern, heterocyclischen Kupplern und Additionssalzen davon ausgewählt ist bzw. sind.

11. Zusammensetzung nach einem der vorhergehenden Ansprüche, wobei es sich bei dem Polyol bzw. den Polyolen um ein $C_4$- $C_{15}$- Polyol bzw. $C_4$- $C_{15}$- Polyole handelt.

12. Zusammensetzung nach Anspruch 11, wobei das Polyol bzw. die Polyole aus Hexylenglykol, Neopentylglykol und Isoprenglykol ausgewählt ist bzw. sind.

13. Zusammensetzung nach einem der vorhergehenden Ansprüche, wobei das Polyol bzw. die Polyole in Anteilen zwischen 0,1 und 40 Gew.-%, bezogen auf das Gesamtgewicht der Färbezusammensetzung, vorliegt bzw. vorliegen.

14. Verfahren zum Färben von Keratinfasern, **dadurch gekennzeichnet, dass** man eine Zusammensetzung gemäß einem der Ansprüche 1 bis 13 in Gegenwart eines Oxidationsmittels über einen zur Entwicklung der gewünschten Färbung ausreichenden Zeitraum auf die Keratinfasern aufbringt.

15. Verfahren nach Anspruch 14, bei dem man das Oxidationsmittel aus Wasserstoffperoxid, Harnstoffperoxid, Alkalimetallbromaten, Persalzen, Persäuren und Oxidaseenzymen auswählt.

16. Vorrichtung mit mehreren Kompartimenten, in der ein erstes Kompartiment eine Färbezusammensetzung gemäß einem der Ansprüche 1 bis 13 enthält und ein zweites Kompartiment ein Oxidationsmittel enthält.

## Claims

1. Composition for dyeing keratinous fibres, comprising, in a medium appropriate for the dyeing of keratinous fibres:

   • one or more aminopyrazolopyridine oxidation bases of following formula (I):

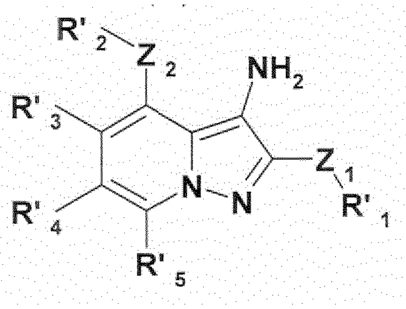

(I)

X

in which:

• $Z_1$ and $Z_2$ independently represent:

- a simple covalent bond,
- a divalent radical chosen from:
- an $-O(CH_2)_p-$ radical, p denoting an integer ranging from 0 to 6,
- an- $NR'_6$ $(CH_2)_q$ $(C_6H_4)_t$- radical, q denoting an integer ranging from 0 to 6, t denoting 0 or 1, and $R'_6$ representing a hydrogen atom or a $C_1$- $C_6$ alkyl radical optionally substituted by one or more hydroxyl groups,

• $Z_1$ can also represent a divalent radical -S-, -SO- or $-SO_2-$ when $R'_1$ is a methyl radical,

• $R'_1$ and $R'_2$ independently represent:

- a hydrogen,
- an optionally substituted $C_1$-$C_{10}$ alkyl radical, optionally interrupted by a heteroatom or a group chosen from O, N, Si, S, SO or $SO_2$,
- a halogen,
- an $SO_3H$ radical,
- a saturated, unsaturated or aromatic and substituted or unsubstituted 5- to 8-membered ring optionally comprising one or more heteroatoms or groups chosen from N, O, S, $SO_2$ or -CO-, it being possible for the ring to be cationic and/or substituted by a cationic radical,
- an- $N^+R_{17}R_{18}R^{19}$ group, $R_{17}$, $R_{18}$ and $R_{19}$ being linear or branched $C_1$- $C_5$ alkyls which are optionally substituted by one or more hydroxyl groups,

when $Z_1$, respectively $Z_2$, represents a covalent bond, then $R'_1$, respectively $R'_2$, can also represent:
- an optionally substituted $C_1$-$C_6$ alkylcarbonyl radical,
- an -O-CO-R, -CO-O-R, NR-CO-R' or -CO-NRR' radical in which R and R' independently represent a hydrogen atom or an optionally substituted $C_1$-$C_6$ alkyl radical,

• $R'_3$, $R'_4$ and $R'_5$, which are identical or different, represent:

- a hydrogen atom,
- a hydroxyl radical,
- a $C_1$-$C_6$ alkoxy radical,
- a $C_1$-$C_6$ alkylthio radical,
- an amino radical,
- a monoalkylamino radical,
- a $C_1$-$C_6$ dialkylamino radical in which the alkyl radicals can form, with the nitrogen atom to which they are attached, a saturated or unsaturated and aromatic or non-aromatic 5- to 8-membered heterocycle which can include one or more heteroatoms or groups chosen from N, O, S, $SO_2$ or CO, it being possible for the heterocycle to be cationic and/or substituted by a cationic radical,
- an optionally substituted $C_1$-$C_6$ alkylcarbonyl radical,
- an -O-CO-R, -CO-O-R, NR-CO-R' or -CO-NRR' radical with R and R' as defined above,
- a halogen,
- an $-NHSO_3H$ radical,
- an optionally substituted $C_1$-$C_4$ alkyl radical,
- a saturated, unsaturated or aromatic and optionally substituted carbon ring,
- it being possible for $R'_3$, $R'_4$ to $R'_5$ to form, in pairs, a ring which is or is not saturated,

• X represents an ion or group of ions which makes it possible to provide the electronegativity of the derivative of formula (I),

with the condition that at least one of the groups $R'_1$ and $R'_2$ represents a cationic radical,

• one or more couplers,

• one or more $C_4$-$C_{30}$ polyols containing a saturated or unsaturated, linear, branched or cyclic hydrocarbon chain which carries at least two hydroxyl functions, the said chain and its branches being optionally substituted by one or more other substituents selected from carboxyl, amino, halogen and $C_6$-$C_{30}$ aryl groups; one or more polyethylene glycols having 4, 6 or 7 ethylene units and dipropylene glycol.

2. Composition according to Claim 1, **characterized in that**, in the formula (I), $Z_1$ and/or $Z_2$ represents a covalent bond, an- $NR'_6$ $(CH_2)_q$- radical or an- O $(CH_2)_p$- radical and $R'_1$ and/or $R'_2$ is a cationic radical.

3. Composition according to Claim 2, in which $R'_1$ or $R'_2$ denote imidazoles substituted by a quaternary ammonium radical or imidazoliums, piperazines substituted by a quaternary ammonium radical or piperaziniums, pyrrolidines substituted by a quaternary ammonium radical or pyrrolidiniums, or diazepanes substituted by a quaternary ammonium radical or diazepaniums.

4. Composition according to Claim 1 or Claim 2, in which $R'_1$ and $R'_2$ independently represent a hydrogen atom or a trialkylammonium, tri (hydroxyalkyl) ammonium, (hydroxyalkyl) dialkylammonium or di (hydroxyalkyl) alkylammonium group.

5. Composition according to any one of the preceding claims, in which the $R'_3$, $R'_4$ and $R'_5$ radicals independently represent a hydrogen atom or an optionally substituted $C_1$-$C_4$ alkyl radical.

6. Composition according to any one of Claims 1 to 5, in which the compound of formula (I) corresponds to the following formula (I'):

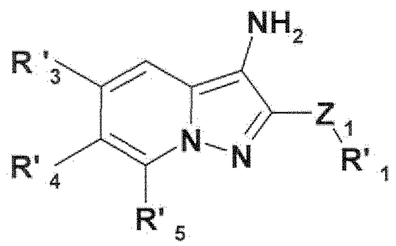

(I')

in which $Z_1$, $R'_1$, $R'_3$, $R'_4$ and $R'_5$ are as defined in any one of Claims 1 to 5.

7. Composition according to Claim 6, in which $Z_1$ represents a covalent bond, an- $NR'_6$ $(CH_2)_q$- radical or an- C $(CH_2)_p$- radical and $R'_1$ is a cationic radical.

8. Composition according to any one of Claims 1 to 7, in which the compound of formula (I) is chosen from:

| |
|---|
| 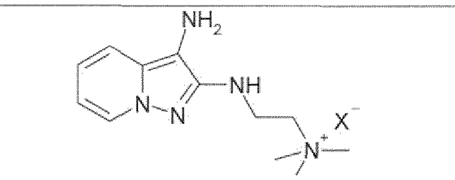 [2-(3-Aminopyrazolo[1,5-a]pyridin-2-ylamino)ethyl]trimethylammonium salt |
| 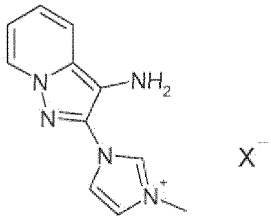 3-(3-Aminopyrazolo[1,5-a]pyridin-2-yl)-1-methyl-3H-imidazol-1-ium salt |
| 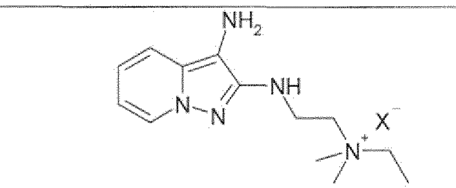 [2-(3-Aminopyrazolo[1,5-a]pyridin-2-ylamino)ethyl]ethyldimethylammonium salt |
| 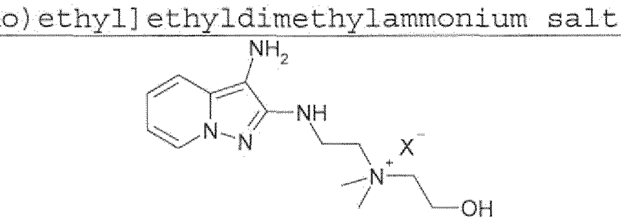 [2-(3-Aminopyrazolo[1,5-a]pyridin-2-ylamino)ethyl](2-hydroxyethyl)dimethylammonium salt |
| 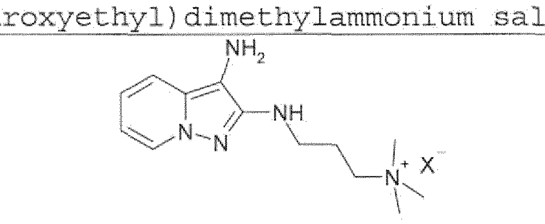 [3-(3-Aminopyrazolo[1,5-a]pyridin-2-ylamino)propyl]trimethylammonium salt |
| 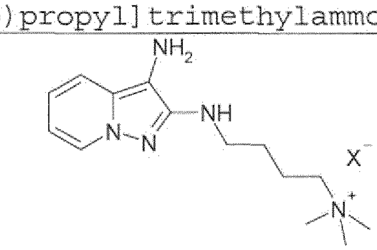 [4-(3-Aminopyrazolo[1,5-a]pyridin-2-ylamino)butyl]-trimethylammonium salt |

| |
|---|
| 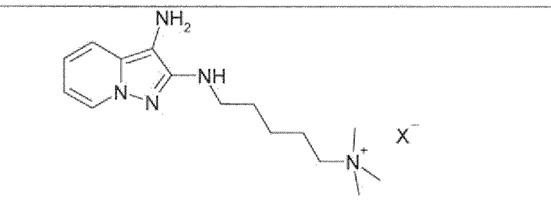<br>[5-(3-Aminopyrazolo[1,5-a]pyridin-2-ylamino)pentyl]trimethylammonium salt |
| 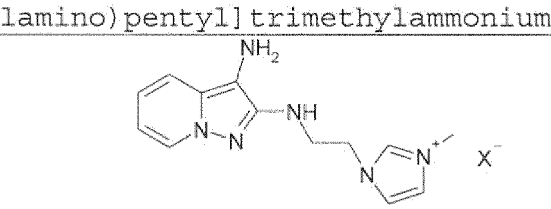<br>3-[2-(3-Aminopyrazolo[1,5-a]pyridin-2-ylamino)ethyl]-1-methyl-3H-imidazol-1-ium salt |
| 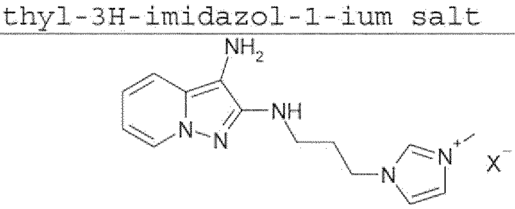<br>3-[3-(3-Aminopyrazolo[1,5-a]pyridin-2-ylamino)propyl]-1-methyl-3H-imidazol-1-ium salt |
| 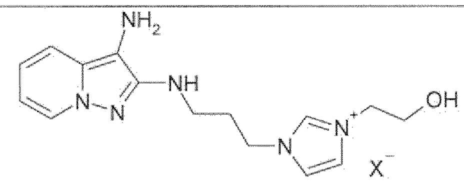<br>3-[3-(3-Aminopyrazolo[1,5-a]pyridin-2-ylamino)propyl]-1-(2-hydroxyethyl)-3H-imidazol-1-ium salt |
| 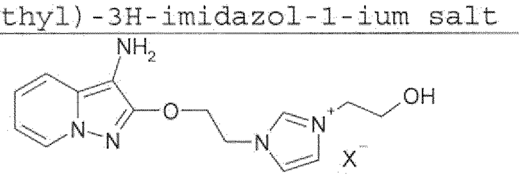<br>3-[2-(3-Aminopyrazolo[1,5-a]pyridin-2-yloxy)ethyl]-1-(2-hydroxyethyl)-3H-imidazol-1-ium salt |
| 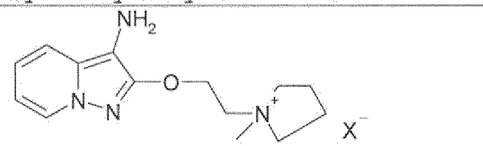<br>1-{2-[(3-Aminopyrazolo[1,5-a]pyridin-2-yl)oxy]ethyl}-1-methylpyrrolidinium salt |
| 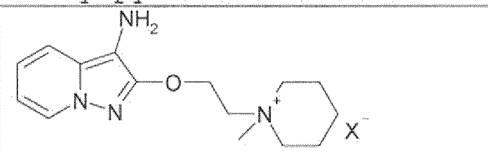 |

| |
|---|
| 1-{2-[(3-Aminopyrazolo[1,5-a]pyridin-2-yl)oxy]ethyl}-1-methylpiperidinium salt |
| 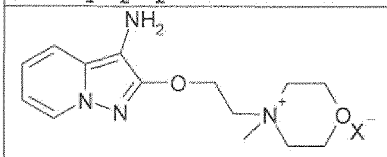 |
| 4-{2-[(3-Aminopyrazolo[1,5-a]pyridin-2-yl)oxy]ethyl}-4-methylmorpholin-4-ium salt |
| 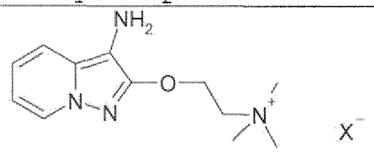 |
| {2-[(3-Aminopyrazolo[1,5-a]pyridin-2-yl)oxy]ethyl}trimethylammonium salt |
| 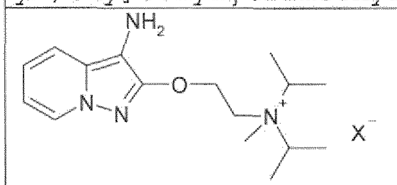 |
| {2-[(3-Aminopyrazolo[1,5-a]pyridin-2-yl)oxy]ethyl}diisopropylmethylammonium salt |
| 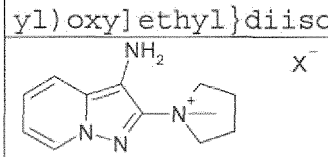 |
| 1-(3-Aminopyrazolo[1,5-a]pyridin-2-yl)-1-methylpyrrolidinium salt |
| 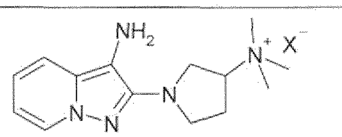 |
| [1-(3-Aminopyrazolo[1,5-a]pyridin-2-yl)pyrrolidin-3-yl]trimethylammonium salt |
| 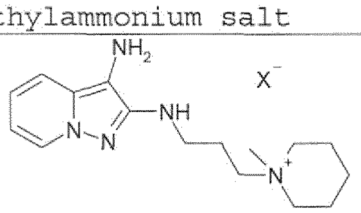 |
| 1-[3-(3-Aminopyrazolo[1,5-a]pyridin-2-ylamino)propyl]-1-methylpiperidinium salt |
| 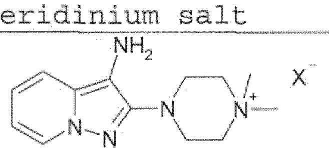 |
| 4-(3-Aminopyrazolo[1,5-a]pyridin-2-yl)-1,1- |

| |
|---|
| dimethylpiperazin-1-ium salt |
| 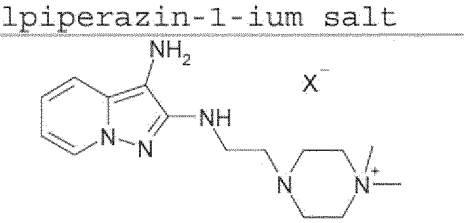 |
| 4-[2-(3-Aminopyrazolo[1,5-a]pyridin-2-ylamino)ethyl]-1,1-dimethylpiperazin-1-ium salt |
| 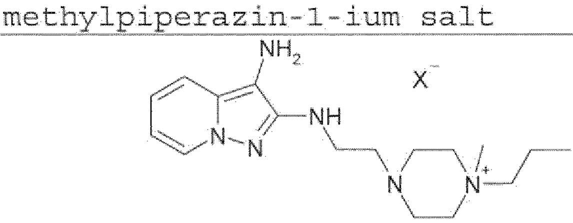 |
| 4-[2-(3-Aminopyrazolo[1,5-a]pyridin-2-ylamino)ethyl]-1-methyl-1-propylpiperazin-1-ium salt |
| 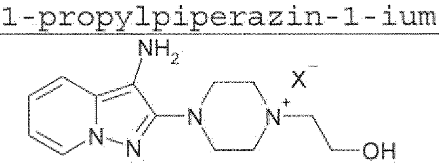 |
| 4-(3-Aminopyrazolo[1,5-a]pyridin-2-yl)-1-(2-hydroxyethyl)piperazin-1-ium salt |
| 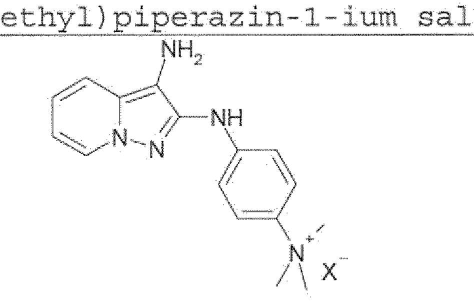 |
| [4-(3-Aminopyrazolo[1,5-a]pyridin-2-ylamino)phenyl]trimethylammonium salt |
| 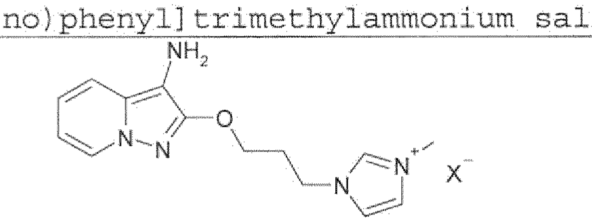 |
| 3-[3-(3-Aminopyrazolo[1,5-a]pyridin-2-yloxy)propyl]-1-methyl-3H-imidazol-1-ium salt |
| 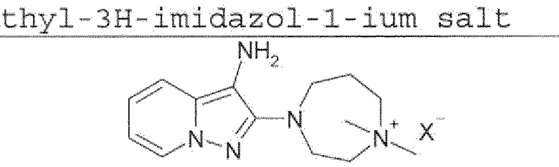 |
| 4-(3-Aminopyrazolo[1,5-a]pyridin-2-yl)-1,1-dimethyl-1,4-diazepan-1-ium salt |

| |
|---|
| 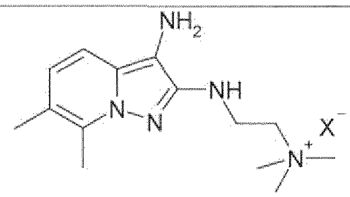<br>[2-(3-Amino-6,7-dimethylpyrazolo[1,5-a]pyridin-2-ylamino)ethyl]trimethylammonium salt |
| 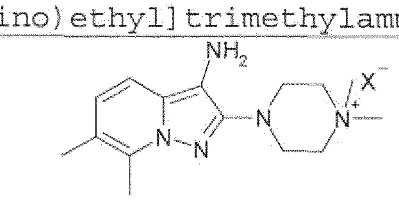<br>4-(3-Amino-6,7-dimethylpyrazolo[1,5-a]pyridin-2-yl)-1,1-dimethylpiperazin-1-ium salt |
| 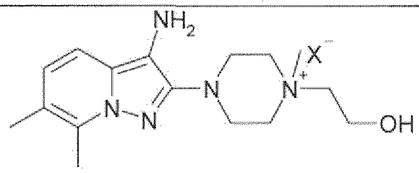<br>4-(3-Amino-6,7-dimethylpyrazolo[1,5-a]pyridin-2-yl)-1-(2-hydroxyethyl)-1-methylpiperazin-1-ium salt |
| 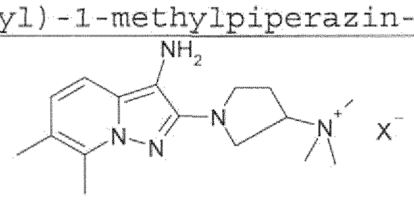<br>[1-(3-Amino-6,7-dimethylpyrazolo[1,5-a]pyridin-2-yl)pyrrolidin-3-yl]trimethylammonium salt |
| 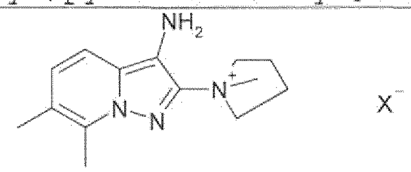<br>1-(3-Amino-6,7-dimethylpyrazolo[1,5-a]pyridin-2-yl)-1-methylpyrrolidinium salt |
| 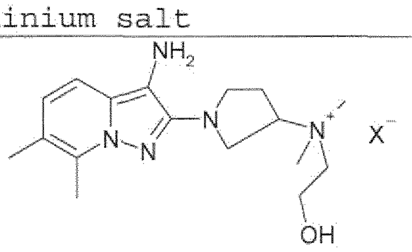<br>[1-(3-Amino-6,7-dimethylpyrazolo[1,5-a]pyridin-2-yl)pyrrolidin-3-yl](2-hydroxyethyl)dimethylammonium salt |

| |
|---|
| 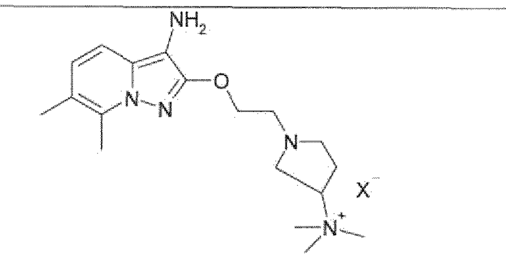<br>{1-[2-(3-Amino-6,7-dimethylpyrazolo[1,5-a]pyridin-2-yloxy)ethyl]pyrrolidin-3-yl}trimethylammonium salt |
| 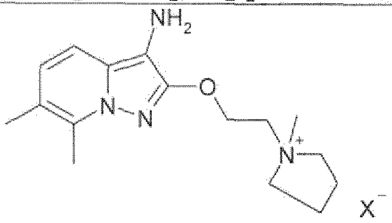<br>1-{2-[(3-Amino-6,7-dimethylpyrazolo[1,5-a]pyridin-2-yl)oxy]ethyl}-1-methylpyrrolidinium salt |
| 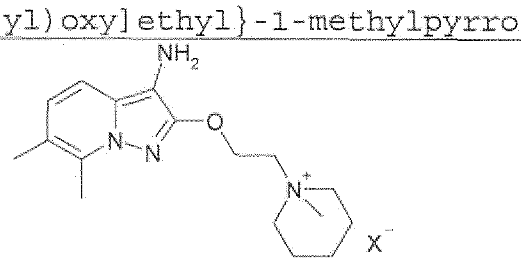<br>1-{2-[(3-Amino-6,7-dimethylpyrazolo[1,5-a]pyridin-2-yl)oxy]ethyl}-1-methylpiperidinium salt |
| 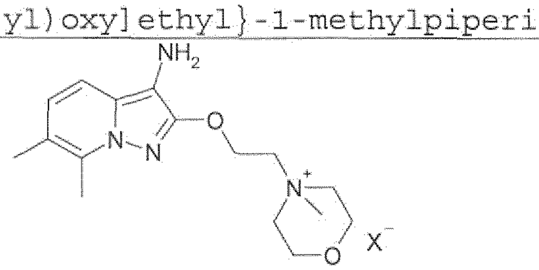<br>4-{2-[(3-Amino-6,7-dimethylpyrazolo[1,5-a]pyridin-2-yl)oxy]ethyl}-4-methylmorpholin-4-ium salt |
| 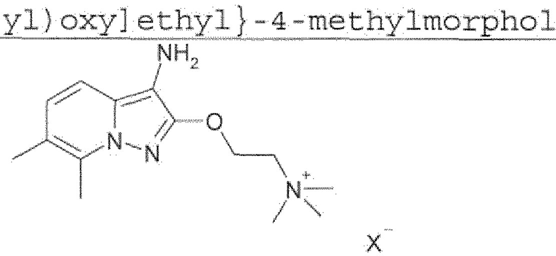<br>{2-[(3-Amino-6,7-dimethylpyrazolo[1,5-a]pyridin-2-yl)oxy]ethyl}trimethylammonium salt |

| |
|---|
| 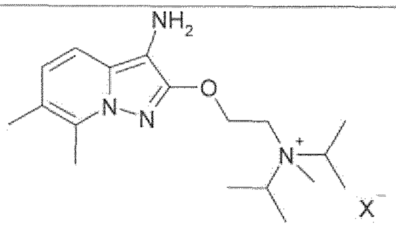<br>{2-[(3-Amino-6,7-dimethylpyrazolo[1,5-a]pyridin-2-yl)oxy]ethyl}diisopropylmethylammonium salt |
| 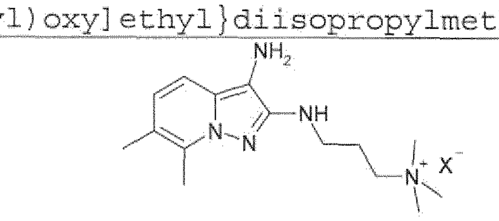<br>[3-(3-Amino-6,7-dimethylpyrazolo[1,5-a]pyridin-2-ylamino)propyl]trimethylammonium salt |
| 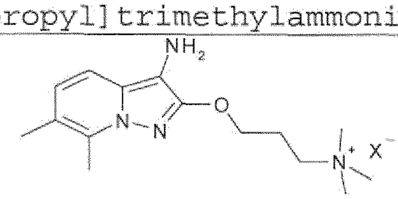<br>[3-(3-Amino-6,7-dimethylpyrazolo[1,5-a]pyridin-2-yloxy)propyl]trimethylammonium salt |
| 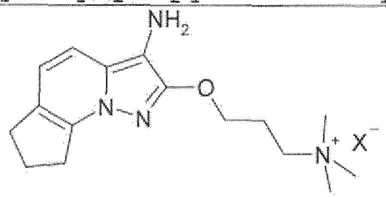<br>[3-(3-Amino-7,8-dihydro-6H-cyclopenta[e]pyrazolo[1,5-a]pyridin-2-yloxy)propyl]trimethylammonium salt |
| 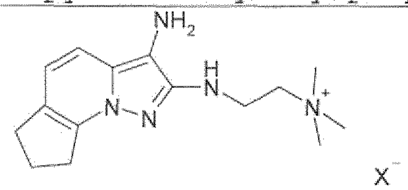<br>{2-[(3-Amino-7,8-dihydro-6H-cyclopenta[e]pyrazolo[1,5-a]pyridin-2-yl)amino]ethyl}trimethylammonium salt |
| 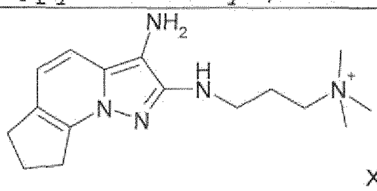<br>{3-[(3-Amino-7,8-dihydro-6H-cyclopenta[e]pyrazolo[1,5-a]pyridin-2-yl)amino]propyl}trimethylammonium salt |

| |
|---|
| 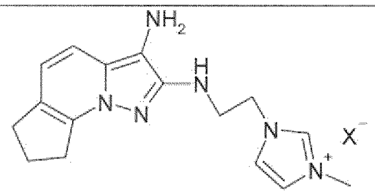<br>1-{2-[(3-Amino-7,8-dihydro-6H-cyclopenta[e]pyrazolo[1,5-a]pyridin-2-yl)amino]ethyl}-3-methyl-1H-imidazol-3-ium salt |
| 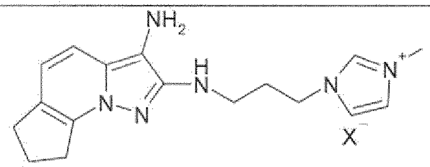<br>1-{3-[(3-Amino-7,8-dihydro-6H-cyclopenta[e]pyrazolo[1,5-a]pyridin-2-yl)amino]propyl}-3-methyl-1H-imidazol-3-ium salt |
| 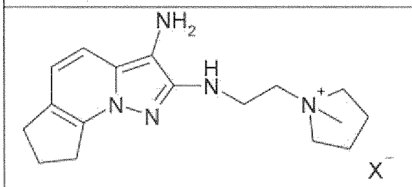<br>1-{2-[(3-Amino-7,8-dihydro-6H-cyclopenta[e]pyrazolo[1,5-a]pyridin-2-yl)amino]ethyl}-1-methylpyrrolidinium salt |
| 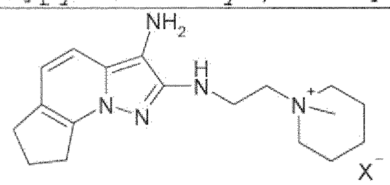<br>1-{2-[(3-Amino-7,8-dihydro-6H-cyclopenta[e]pyrazolo[1,5-a]pyridin-2-yl)amino]ethyl}-1-methylpiperidinium salt |
| 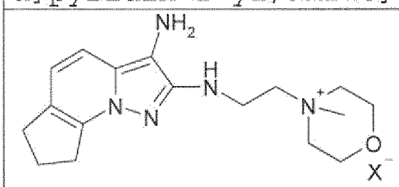<br>4-{2-[(3-Amino-7,8-dihydro-6H-cyclopenta[e]pyrazolo[1,5-a]pyridin-2-yl)amino]ethyl}-4-methylmorpholin-4-ium salt |
| 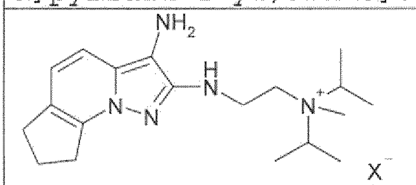<br>{2-[(3-Amino-7,8-dihydro-6H-cyclopenta[e]pyrazolo[1,5-a]pyridin-2-yl)amino]ethyl}diisopropylmethylammonium salt |

| |
|---|
| 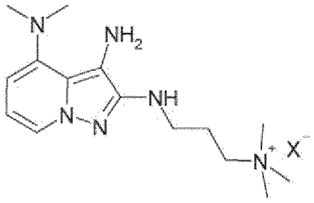<br>[3-(3-Amino-4-(dimethylamino)pyrazolo[1,5-a]pyridin-2-ylamino)propyl]trimethylammonium salt |
| 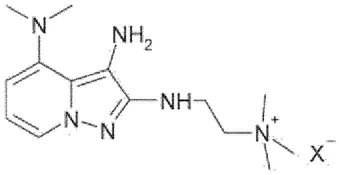<br>[2-(3-Amino-4-(dimethylamino)pyrazolo[1,5-a]pyridin-2-ylamino)ethyl]trimethylammonium salt |
| 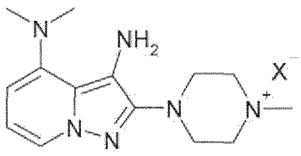<br>4-(3-Amino-4-(dimethylamino)pyrazolo[1,5-a]pyridin-2-yl)-1-methylpiperazin-1-ium salt |
| 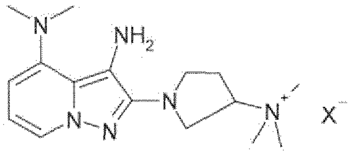<br>[1-(3-Amino-4-(dimethylamino)pyrazolo[1,5-a]pyridin-2-yl)pyrrolidin-3-yl]trimethylammonium salt |
| 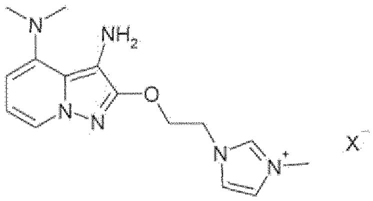<br>3-[2-(3-Amino-4-(dimethylamino)pyrazolo[1,5-a]pyridin-2-yloxy)ethyl]-1-methyl-3H-imidazol-1-ium salt |
| 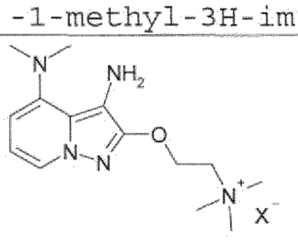<br>[2-(3-Amino-4-(dimethylamino)pyrazolo[1,5-a]pyridin-2-yloxy)ethyl]trimethylammonium salt |

| |
|---|
| 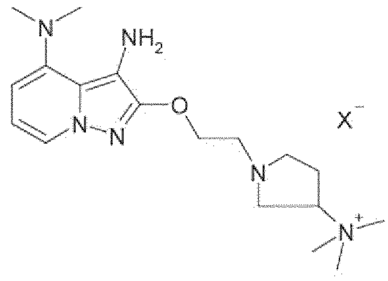 {1-[2-(3-Amino-4-(dimethylamino)pyrazolo[1,5-a]pyridin-2-yloxy)ethyl]pyrrolidin-3-yl}trimethylammonium salt |
| 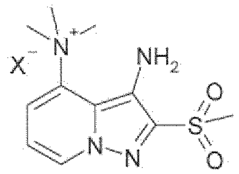 (3-Amino-2-(methanesulfonyl)pyrazolo[1,5-a]pyridin-4-yl)-trimethylammonium salt |
| 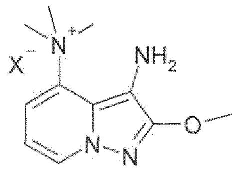 (3-Amino-2-methoxypyrazolo[1,5-a]pyridin-4-yl)trimethylammonium salt |

**9.** Composition according to any one of the preceding claims, **characterized in that** the content of compound of formula (I) is between 0.001 and 10% by weight approximately of the total weight of the dyeing composition.

**10.** Composition according to any one of the preceding claims, **characterized in that** the coupler or couplers are chosen from meta-phenylenediamines, meta-aminophenols, meta-diphenols, naphthalene couplers, heterocyclic couplers and their addition salts.

**11.** Composition according to any one of the preceding claims, in which the polyol or polyols are $C_4$-$C_{15}$ polyols.

**12.** Composition according to Claim 11, in which the polyol or polyols are selected from hexylene glycol; neopentyl glycol; and isoprene glycol.

**13.** Composition according to any one of the preceding claims, in which the polyol or polyols are present in proportions of between 0.1 and 40% by weight of the total weight of the dyeing composition.

**14.** Method for dyeing keratinous fibres, **characterized in that** a composition as defined in any one of Claims 1 to 13 is applied to the keratinous fibres in the presence of an oxidizing agent for a time sufficient to develop the desired colouring.

**15.** Method according to Claim 14, in which the oxidizing agent is chosen from hydrogen peroxide, urea hydrogen peroxide, alkali metal bromates, persalts, peracids and oxidase enzymes.

**16.** Multicompartment device, in which a first compartment comprises a dyeing composition as defined in any one of Claims 1 to 13 and a second compartment comprises an oxidizing agent.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2801308
- FR 2886139
- GB 1026978 A
- GB 1153196 A
- DE 2359399
- JP 63169571 A
- JP 5063124 A
- EP 0770375 A
- WO 9615765 A
- FR 2750048 A
- DE 3843892
- DE 4133957
- WO 9408969 A
- WO 9408970 A
- FR 2733749 A
- DE 19543988
- FR 2886137
- FR 2586913